# EUROPEAN PATENT APPLICATION

(11) **EP 4 471 051 A1**
(43) Date of publication of application: **04.12.2024**
(21) Application number: 23746402.9
(22) Date of filing: 28.01.2023
(51) Int. Cl.: C07K 14/31, C07K 19/00, C07K 16/00, C07K 1/22, C12N 15/31, C12N 15/62, B01D 15/38

(54) **B DOMAIN AND Z DOMAIN MUTANTS OF PROTEIN A, AND APPLICATION THEREOF**

(30) Priority: 28.01.2022 CN 202210108116
(71) Applicant: Bestchrom (Zhejiang) Biosciences Ltd., Zhejiang 314299 (CN); Bestchrom (Shanghai) Biosciences Ltd., Shanghai 201203 (CN)
(72) Inventor: ZHANG, Hong, Jiaxing, Zhejiang 314299 (CN); SHI, Haitao, Jiaxing, Zhejiang 314299 (CN); ZHANG, Yan, Jiaxing, Zhejiang 314299 (CN); HU, Huixia, Jiaxing, Zhejiang 314299 (CN)
(74) Representative: V.O.
(86) International application number: PCT/CN2023/073587
(87) International publication number: WO 2023/143525

(57) **Abstract**

B domain and Z domain mutants of a protein A, and an application thereof. Specifically, provided is an isolated polypeptide, which is selected from: (1) a polypeptide having a substitution mutation at one or more positions selected from positions 3, 6, 9, 15 and 23 compared with the native B domain of protein A shown in SEQ ID NO: 1 or the Z domain of protein A shown in SEQ ID NO: 2; and (2) a polypeptide having at least 85% sequence identity with the polypeptide described in (1) and retaining the substitution mutation at one or more of the positions 3, 6, 9, 15 and 23. Also provided are a fusion protein or protein A comprising the polypeptide, and a separation matrix. The fusion protein or protein A comprising the polypeptide has significantly improved alkaline stability during alkaline cleaning.

## Description

### FIELD OF THE INVENTION

The invention relates to B domain and Z domain mutants of protein A, and application thereof.

### BACKGROUND OF THE INVENTION

In recent years, antibody drugs are gaining increasing popularity in the global pharmaceutical market due to their high specificity, and monoclonal antibodies are one of the fastest growing fields in the biopharmaceutical industry. The higher the purity of the monoclonal antibody, the better the effect and the higher the efficiency. The research on the purification of various antibody drugs such as monoclonal antibody has always been the focus of research in the current field. Protein A affinity chromatography media has become the most widely used method for purifying mAbs due to its special adsorption capacity for the unique Fc fragment of antibodies. However, the protein A affinity chromatography media may have problems such as ligand shedding and low binding capacity during antibody purification. In addition, 0.5-1.0 M NaOH solution is used in the cleaning-in-place (CIP) process in the purification procedure. High concentrations of NaOH will destroy the Protein A affinity chromatography media and greatly reduce its service life. Therefore, researchers have conducted a lot of research on how to improve the alkali resistance of Protein A. For example, Susanne Gilich et al. proposed that protein engineering can be used to improve the stability of protein ligand domains under alkaline conditions. To this end, those skilled in the art conduct mutation research on amino acids in various domains of protein ligands to find amino acids that can improve the alkali resistance of protein ligands.

Protein A is composed of highly homologous immunoglobulin binding domains E, D, A, B, and C, wherein domain B has a greater advantage in binding specificity to immunoglobulins. Domain Z is obtained by replacing alanine at position 1 with valine and replacing glycine at position 29 with alanine in domain B of Protein A. This mutation endows domain Z with more prominent chemical stability, and similarly, domain Z mutated by the amino acid of the domain has certain stability under alkaline conditions. However, it is still unstable at higher pH and cannot meet the requirements of the CIP wash step in the antibody purification process. Compared with the native Protein A chromatography media, the existing Protein A chromatography media with B domain or Z domain mutation has certain alkaline stability. For example, CN101522278A mutates glycine at position 29 in the B domain to alanine, thereby improving alkali resistance. CN1642976A discloses the mutation of at least one asparagine residue in the Z domain to an amino acid other than alanine, threonine or aspartic acid. In addition, CN105377881A discloses that at least the glutamic acid residue at position 15 in the B domain or the Z domain is mutated to amino acids other than asparagine or glutamine. These mutants have higher alkali tolerance under alkaline conditions compared to the native ligands. The chromatography media prepared by using the above ligands can withstand 0.1-0.5 M sodium hydroxide washing in the antibody purification process, but there is still a certain distance from the standard CIP (0.5-1.0 M sodium hydroxide).

### SUMMARY OF THE INVENTION

The first aspect of the present description provides an isolated polypeptide selected from the group consisting of:
(1)compared with the native B domain of protein A shown in SEQ ID NO: 1 or the Z domain of protein A shown in SEQ ID NO: 2, the polypeptide has substitution mutation at one or more positions selected from positions 3, 6, 9, 15 and 23;
   wherein, the substitution mutation at position 3 is that asparagine is replaced by leucine, isoleucine, valine or tyrosine; the substitution mutation at position 6 is that asparagine is replaced by glutamic acid, leucine, isoleucine, valine, serine or threonine; the substitution mutation at position 9 is that glutamine is replaced by isoleucine, leucine, valine, phenylalanine or methionine; the substitution mutation at position 15 is that glutamic acid is replaced by threonine, tryptophan, leucine, valine, isoleucine, phenylalanine, serine, tyrosine or aspartic acid; the substitution mutation at position 23 is that asparagine is replaced by valine, isoleucine, leucine or tyrosine;
(2) has at least 85% identity with the polypeptide described in (1) and retains the substitution mutation at one or more positions of position 3, 6, 9, 15 and 23.

In one or more embodiments, the substitution mutation at position 3 is that asparagine is replaced by leucine, valine or tyrosine.

In one or more embodiments, the substitution mutation at position 6 is that asparagine is replaced by glutamic acid, leucine or serine.

In one or more embodiments, the substitution mutation at position 9 is that glutamine is replaced by isoleucine, phenylalanine, or methionine.

In one or more embodiments, the substitution mutation at position 15 is that glutamate is replaced by threonine, tryptophan, leucine, isoleucine, valine, phenylalanine, serine, or tyrosine acid.

In one or more embodiments, the substitution mutation at position 23 is that asparagine is replaced by valine, isoleucine, or tyrosine.

In one or more embodiments, the polypeptide has at least the substitution mutation at position 3, and has the substitution mutation at least at one position, at least at two positions, at least at three positions, or at all four positions selected from the group consisting of positions 15, 6, 9, and 23.

In one or more embodiments, the polypeptide has at least the substitution mutation at position 6, and has the substitution mutation at least at one position, at least at two positions, at least at three positions, or at all four positions selected from the group consisting of positions 3, 15, 9, and 23.

In one or more embodiments, the polypeptide has at least the substitution mutation at position 9, and has the substitution mutation at least at one position, at least at two positions, at least at three positions, or at all four positions selected from the group consisting of positions 3, 6, 15, and 23.

In one or more embodiments, the polypeptide has at least the substitution mutation at position 15, and has the substitution mutation at least at one position, at least at two positions, at least at three positions, or at all four positions selected from the group consisting of positions 3, 6, 9, and 23.

In one or more embodiments, the polypeptide has at least the substitution mutation at position 23, and has the substitution mutation at least at one position, at least at two positions, at least at three positions, or at all four positions selected from the group consisting of positions 3, 6, 9, and 15.

In one or more embodiments, the substitution mutation at position 15 is E151, E15L or E15V, the substitution mutation at position 3 is N3L or N3V, the substitution mutation at position 6 is N6E or N6L, and the substitution mutation at position 9 is Q91 or Q9F, and the substitution mutation at position 23 is N231, N23Y or N23V.

In one or more embodiments, the polypeptide has the substitution mutations at positions 3, 9 and 15, and has the substitution mutations at positions 6 and/or 23; preferably, the substitution mutation at position 3 is N3L or N3V, the substitution mutation at position 6 is N6L or N6E, the substitution mutation at position 9 is Q91 or Q9F, and the substitution mutation at position 15 is E15L, E151 or E15V, the substitution mutation at position 23 is N23V, N231 or N23Y.

In one or more embodiments, the polypeptide has the substitution mutations at positions 3, 6, 9 and 15. Preferably, the substitution mutation at position 3 is N3L or N3V, preferably N3V, the substitution mutation at position 6 is N6L or N6E, preferably N6E, the substitution mutation at position 9 is Q91 or Q9F, preferably Q91, the substitution mutation at position 15 is E15L, E151 or E15V, preferably E15L.

In one or more embodiments, the amino acid sequence of the polypeptide is as shown in any one of SEQ ID NO: 3-71 and SEQ ID NO: 118-124.

The second aspect of the present description provides an isolated polypeptide, which consists of the following (i), (ii) and (iii): (i) the polypeptide described in any embodiment of the first aspect of the present description, (ii) one or more coupling elements at the C-terminus or N-terminus of the amino acid sequence of the polypeptide (i), and optionally (iii) residues from the excised signal transduction sequence.

In one or more embodiments, the coupling elements are selected from the group consisting of: cysteine residues, multiple lysine residues and multiple histidine residues; the residues from the excised signal transduction sequence is AQ.

The third aspect of the present description provides a fusion protein, wherein the fusion protein comprises an amino acid sequence formed by fusion of 2-8 polypeptides according to any embodiment of the first aspect of the present description, wherein the 2-8 polypeptides are different from each other, partially or completely identical, and optionally there is a linker sequence between the polypeptides.

In one or more embodiments, at least one polypeptide of the 2-8 polypeptides contained in the fusion protein has the substitution mutation at position 15, optionally has the substitution mutation at least at one position, at least at two positions, at least at three positions or at all four positions selected from the group consisting of positions 3, 6, 9 and 23; preferably, at least one polypeptide of the 2-8 polypeptides contained in the fusion protein has the substitution mutation at position 3, 9, 15, and has the substitution mutation at position 6 and/or 23.

In one or more embodiments, in the fusion protein, the polypeptide is selected from one or more of: SEQ ID NO: 4, SEQ ID NO: 8, SEQ ID NO: 9, SEQ ID NO: 10, SEQ ID NO: 11, SEQ ID NO: 14, SEQ ID NO: 16, SEQ ID NO: 18, SEQ ID NO: 21, SEQ ID NO: 22, SEQ ID NO: 51, SEQ ID NO: 53, SEQ ID NO: 60, SEQ ID NO: 71, SEQ ID NO: 64, SEQ ID NO: 69, SEQ ID NO: 70, SEQ ID NO: 68, SEQ ID NO: 118, SEQ ID NO: 119, SEQ ID NO: 120, SEQ ID NO: 121, SEQ ID NO: 122, SEQ ID NO: 123, and SEQ ID NO: 124.

In one or more embodiments, the C-terminus or N-terminus of the fusion protein further comprises one or more coupling elements and/or residues from the excised signal transduction sequences; preferably, the coupling elements are selected from the group consisting of: cysteine residues, multiple lysine residues, and multiple histidine residues.

In one or more embodiments, the amino acid sequence of the fusion protein is as shown in any one of SEQ ID NO: 73-109 and SEQ ID NO: 111-117.

The fourth aspect of the present description provides a recombinant protein A, the B domain of the recombinant protein A is the polypeptide according to any embodiment of the first aspect of the present description.

The fifth aspect of the present description provides an isolated nucleic acid molecule, the polynucleotide sequence of the nucleic acid molecule is selected from:
(1) a polynucleotide sequence encoding the polypeptide, fusion protein or recombinant protein A according to any embodiment of the present description; (2) the complementary sequence of the polynucleotide sequence of (1).

The sixth aspect of the present description provides a nucleic acid construct, the nucleic acid construct comprises the nucleic acid molecule according to any embodiment of the present description; preferably, the nucleic acid construct is an expression cassette; more preferably, the nucleic acid construct is an expression vector or a cloning vector.

The seventh aspect of the present description provides an expression system comprising the nucleic acid construct according to any embodiment of the present description; preferably, the expression system is a host cell.

The eighth aspect of the present description provides a separation medium comprising the polypeptide, fusion protein and/or recombinant protein A according to any embodiment of the present description, coupled to a solid support.

In one or more embodiments, the polypeptide, the fusion protein or the recombinant protein A is coupled to the solid support through a thioether bond.

In one or more embodiments, the solid support is selected from: polyhydroxy-containing polymers, preferably polysaccharides, more preferably selected from: dextran, starch, cellulose, pullulan, agar and agarose; synthetic polymers, preferably selected from the group consisting of: polyvinyl alcohol, polystyrene, polystyrenedivinylbenzene, polyhydroxyalkyl acrylates, polyhydroxyalkyl methacrylates, polyacrylamides and polymethyl methacrylates acrylamide; and a inorganic support, preferably selected from silica and zirconia.

The ninth aspect of the present description provides a chromatographic column, which comprises the separation medium according to any embodiment of the present description.

A tenth aspect of the present description provides a method for separating Fc-containing proteins, comprising a step where a sample containing immunoglobulin comes in contact with the polypeptide, fusion protein, recombinant protein A, separation medium or chromatography column according to any of the embodiments of the present description; preferably, the Fc-containing protein is immunoglobulin.

In one or more embodiments, the method comprises: (1) contacting the sample containing the Fc-containing protein with the separation medium ; (2) washing the separation medium; (3) eluting the Fc-containing protein from the separation medium ; (4) washing the separation medium.

In one or more embodiments, washing the separation medium with a 0.1-2.0 M or 0.5-1.0 M NaOH or KOH solution.

The eleventh aspect of the present invention provides the use of the polypeptide, fusion protein or protein A according to any embodiment of the present description in separating Fc-containing proteins, or in the preparation of a separation medium or chromatographic column for separating Fc-containing proteins.

### Description of drawings

Figure 1: gene result validation analysis diagram of (B domain)4.
Figure 2: gene results validation analysis diagram of mutant B(N3V)4.
Figure 3: gene results validation analysis diagram of mutant B(N6E)4.
Figure 4: gene results validation analysis diagram of mutant B(Q91)4.
Figure 5: gene results validation analysis diagram of mutant B Q9F)4.
Figure 6: gene results validation analysis of mutant B(E15L)4.
Figure 7: gene results validation analysis diagram of mutant B(E151)4.
Figure 8: gene results validation analysis diagram of mutant B(N3V, N6E, Q9I, E15L)4.
Figure 9: gene result validation analysis diagram of (Z domain)4.
Figure 10: gene results validation analysis diagram of mutant Z Q9I)4.
Figure 11: gene results validation analysis diagram of mutant Z(E15L)4.
Figure 12: gene results validation analysis diagram of mutant Z(E151)4.
Figure 13: gene results validation analysis diagram of mutant Z(N3 V, N6E, Q9I, E15L)4.
Figure 14: the alkali-resistant stability curve of the Z domain mutant shown in SEQ ID NO: 72-91.
Figure 15: the alkali-resistant stability curves of the Z domains shown in SEQ ID NO: 72, 98-102, 104, 105 and 107-109.

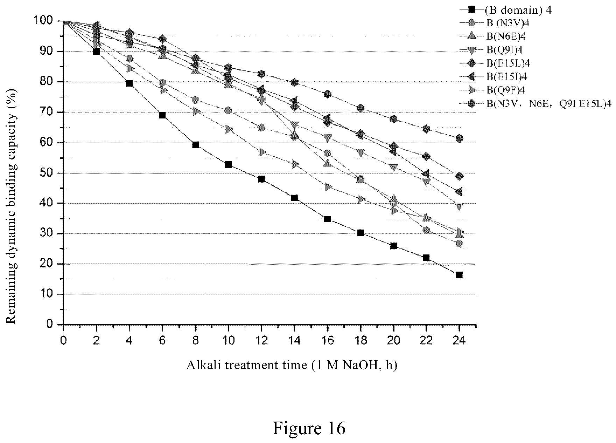

Figure 16: the alkali-resistant stability curve of the B domain mutant shown in SEQ ID NO: 110-117.

### DETAILED DESCRIPTION

It should be understood that within the scope of the present description, the above-mentioned technical features of the present description and the technical features specifically described in the following (such as the examles) can be combined with each other to form a preferred technical solution.

Herein, "antibody" and "immunoglobulin" are used interchangeably and have meanings well known in the art. Antibodies or immunoglobulins as described herein also comprise fragments of antibodies, and fusion proteins or conjugates containing fragments of antibodies, provided such Fc-containing antibody fragments, fusion proteins or conjugates can be separated and purified via binding with protein A. Fragments of an antibody may be functionally active fragments thereof.. Fragments of an antibody may be functionally active fragments thereof.

In this specification, amino acid residues also use following abbreviations: alanine (Ala or A), arginine (Arg or R), asparagine (Asn or N), aspartic acid (Asp or D) ), cysteine (Cys or C), glutamine (Gln or Q), glutamic acid (Glu or E), glycine (Gly or G), histidine (His or H), isoleucine (Ile or 1), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), valine (Val or V), and any amino acid residue (Xaa or X). In addition, in this specification, the amino acid sequence of a peptide is described so that the amino terminus (hereinafter referred to as N-terminus) is on the left side and the carboxy-terminus (hereinafter referred to as C-terminus) is on the right side according to usual practice.

Protein A is widely used as an affinity medium ligand for the purification of immunoglobulins. Native protein A has five domains binding with immunoglobulins, especially IgG, which are E domain, D domain, A domain, B domain and C domain from the N-terminus to C-terminus, respectively. Domain Z is obtained by replacing alanine at position 1 with valine and replacing glycine at position 29 with alanine in domain B of protein A. This mutation confers greater chemical stability to domain Z. In the process of immunoglobulin purification, the immunoglobulin needs to be eluted with alkali after combined with protein A. This process will cause protein A to shed from the affinity chromatography medium and cause contamination to the immunoglobulin product.

The present description finds that, when at least one of positions 3, 6, 9 and 15 of the native B domain or Z domain of protein A undergoes substitution mutation, , the resulted mutant of the B or Z domain will witness significantly improved alkaline stability during alkaline cleaning process compared to the native B domain or the unmutated Z domain. The concentration of alkali used can be increased from 0.1-0.5 M to 0.5-2.0 M(such as 0.5-1.0 M). The present description areis based on these facts.

Therefore, the present description provides a polypeptide that has a substitution mutation at least at one position, at least at two positions, at least at three positions, at least at four positions, or at all five positions selected from positions 3, 6, 9, 15, and 23 compared to the native B domain shown in SEQ ID NO: 1, or has a substitution mutation at least at one position, at least at two positions, at least at three positions, at least at four positions, or at all five positions selected from positions 3, 6, 9, 15, and 23 compared to the Z domain shown in SEQ ID NO: 2.

In one or more embodiments, the substitution mutation at position 3 is that asparagine is replaced by leucine, isoleucine, valine or tyrosine. In some embodiments, the substitution mutation at position 3 is that asparagine is replaced by leucine, valine or tyrosine, preferably asparagine is replaced by leucine or valine. In some embodiments, the substitution mutation at position 3 is that asparagine is replaced by valine. In some embodiments, the polypeptide described herein has a substitution mutation at position 3, preferably, the amino acid sequence of the polypeptide is shown in SEQ ID NO: 3, 4, 5 or 118.

In one or more embodiments, the substitution mutation at position 6 is that asparagine is replaced by glutamic acid, leucine, isoleucine, valine, serine or threonine. In some embodiments, the substitution mutation at position 6 is that asparagine is replaced by glutamic acid, leucine or serine. In some embodiments, the substitution mutation at position 6 is that asparagine is replaced by leucine. In some embodiments, the polypeptide described herein has a substitution mutation at position 6, preferably, the amino acid sequence of the polypeptide is shown in SEQ ID NO: 6, 7, 8 or 119.

In one or more embodiments, the substitution mutation at position 9 is that glutamine is replaced by isoleucine, leucine, valine, phenylalanine or methionine. In someembodiments, the substitution mutation at position 9 is that glutamine is replaced by isoleucine, phenylalanine or methionine. In some embodiments, the polypeptide described herein has a substitution mutation at position 9, preferably, the amino acid sequence of the polypeptide is shown in SEQ ID NO: 9, 10, 11, 120 or 121.

In one or more embodiments, the substitution mutation at position 15 is that glutamate is replaced by threonine, tryptophan, leucine, valine, isoleucine, phenylalanine, serine, tyrosine or aspartic acid. In someembodiments, the substitution mutation at position 15 is that glutamate is replaced by threonine, tryptophan, leucine, valine, phenylalanine, serine or tyrosine. In one or more embodiments, the substitution mutation at position 15 is that glutamate is replaced by leucine or serine. In some embodiments, the polypeptide described herein has a substitution mutation at position 15, preferably, the amino acid sequence of the polypeptide is shown in any one of SEQ ID NO: 12-20 and 122, 123.

In one or more embodiments, the substitution mutation at position 23 is that asparagine is replaced by valine, isoleucine, leucine or tyrosine. In someembodiments, the substitution mutation at position 23 is that asparagine is replaced by valine, isoleucine or tyrosine. In some embodiments, the substitution mutation at position 23 is that asparagine is replaced by valine or isoleucine. In some embodiments, the polypeptide described herein has a substitution mutation at position 23, preferably, the amino acid sequence of the polypeptide is shown in any one of SEQ ID NO: 21-23.

In one or more embodiments, the polypeptide of the present description has at least a substitution mutation as described in any one of the embodiments herein at position 3, and optionally has a substitution mutation as described in any one of the embodiments herein at one or more positions selected from positions 6, 9, 15 and 23, preferably at one or more positions selected from positions 6, 9, and 15. In other embodiments, the polypeptide of the present description has at least a substitution mutation as described in any one of the embodiments herein at position 6, and optionally has a substitution mutation as described in any one of the embodiments herein at one or more positions selected from positions 3, 9, 15 and 23, preferably at one or more positions selected from positions 3, 9, and 15. In other embodiments, the polypeptide of the present description has at least a substitution mutation as described in any one of the embodiments herein at position 9, and optionally has a substitution mutation as described in any one of the embodiments herein at one or more positions selected from positions 3, 6, 15 and 23, preferably at one or more positions selected from positions 3, 6, and 15. In other embodiments, the polypeptide of the present description has at least a substitution mutation as described in any one of the embodiments herein at position 15, and optionally has a substitution mutation as described in any one of the embodiments herein at one or more positions selected from positions 3, 6, 9 and 23, preferably at one or more positions selected from positions 3, 6, and 9. In other embodiments, the polypeptide of the present description has at least a substitution mutation as described in any one of the embodiments herein at position 23, and optionally has a substitution mutation as described in any one of the embodiments herein at one or more positions selected from positions 3, 6, 9 and 15. In some embodiments, the substitution mutation at position 3 is N3L or N3V, the substitution mutation at position 6 is N6L or N6E, the substitution mutation at position 9 is Q91 or Q9F, the substitution mutation at position 15 is E15L, E151 or E15V, and the substitution mutation at position 23 is N23V, N231 or N23Y.

In one or more embodiments, the polypeptide has at least the substitution mutation at position 15, and has the substitution mutation as described in any one of the embodiments herein at least at one position, at least at two positions, at least at three positions, or at all four positions selected from positions 3, 6, 9, and 23. In some embodiments, the substitution mutation at position 15 is E15T, E15W, E15L, E15V, E151, E15F, E15S, E15Y or E15T, the substitution mutation at position 3 is N3L, N3V or N3Y, the substitution mutation at position 6 is N6S, N6L or N6E, the substitution mutation at position 9 is Q91 or Q9F, and the substitution mutation at position 23 is N23V, N231 or N23Y. Preferably, the substitution mutation at position 15 is E151 or E15V, the substitution mutation at position 3 is N3L or N3V, the substitution mutation at position 6 is N6E or N6L, and the substitution mutation at position 9 is Q91 or Q9F, the substitution mutation at position 23 is N231, N23Y or N23V.

In other embodiments, the polypeptide of the present description has at least a substitution mutation as described in any one of the embodiments herein at positions 3, 9, 15, and has the substitution mutation as described in any one of the embodiments herein at positions 6 and/or 23. In some embodiments, the substitution mutation at position 15 is E15T, E15W, E15L, E15V, E151, E15F, E15S, E15Y or E15T, the substitution mutation at position 3 is N3L, N3V or N3Y, the substitution mutation at position 6 is N6S, N6L or N6E, the substitution mutation at position 9 is Q91 or Q9F, and the substitution mutation at position 23 is N23V, N231 or N23Y. Preferably, the substitution mutation at position 3 is N3L or N3V, the substitution mutation at position 6 is N6L or N6E, the substitution mutation at position 9 is Q91 or Q9F, and the substitution mutation at position 15 is E15L, E151 or E15V, the substitution mutation at position 23 is N23V, N231 or N23Y.

In some embodiments, compared to SEQ ID NO: 1 or 2, the amino acid sequence of the polypeptide described herein has an N3V mutation at position 3, or has an N6E mutation at position 6, or has a Q91 or Q9F mutation at position 9, or has an E15L or E151 mutation at position 15, or has N3V, N6E, Q9I or Q9F and E15L or E151 mutations at positions 3, 6, 9 and 15.

In some embodiments, the amino acid sequence of a polypeptide described herein having substitution mutations at least at two positions selected from positions 3, 6, 9, 15, and 23 is as shown in any of SEQ ID NOs: 24-71.

The polypeptide of the present description can be directly used as a ligand for binding and separating immunoglobulins. Accordingly, in some embodiments, the polypeptide further comprises one or more coupling elements at its C- or N-terminus for coupling the polypeptide to a solid support. Suitable coupling elements are well known in the art and include, but are not limited to, cysteine residues, multiple lysine residues (such as 3-15 or 5-10) and multiple histidine residues (such as 3-15 or 5-10). The coupling element can be a cysteine residue located at the C-terminus of the polypeptide, allowing the polypeptide to be coupled to the solid support by reaction between the thiol group in the cysteine residue and the electrophilic group on the solid support. The coupling element can be directly connected to the polypeptide, alternatively, it can be attached to the N- or C-terminus of the polypeptide via a linker. The linker may be a linker commonly used in the art, and its presence will not affect the binding activity of the polypeptide. An exemplary linker is a linker sequence comprising or consisting of G and S, typically 2-20 amino acid residues in length. Exemplary linker sequences such as (GS)ₙ, (GSS)ₙ, (GSSS)ₙ and (GSSSS)ₙ, among which n can be an integer ranging from 2 to 10, while the total length of the linker will normally not exceed 20 amino acid residues. The polypeptide may also comprise multiple amino acid residues (e.g., fewer than 15, 10 or 5) at the N-terminus, which are derived from the cloning process or residues from signal transduction sequences. For example, the polypeptide may comprise AQ at the N-terminus.

The present description also provides a fusion protein, which is formed by the fusion of 2-8 aforementioned polypeptides of the present description. The polypeptides forming the fusion protein may be all different, partly or entirely identical. Each polypeptide can be directly connected through the peptide bonds at the C-terminus and N-terminus, respectively. Alternatively, any two polypeptides can also be connected through a linker. The linker may be a linker commonly used in the art, and its presence will not affect the binding activity of the polypeptide. An exemplary linker is a linker sequence comprising or consisting of G and S, typically 2-20 amino acid residues in length. Exemplary linker sequences such as (GS)ₙ and (GSS)ₙ, among which n can be an integer ranging from 2 to 10, while the total length of the linker will normally not exceed 20 amino acid residues.

The fusion protein can be used as a ligand for binding and separating immunoglobulins. Accordingly, in some embodiments, the fusion protein further comprises one or more coupling elements at its C- or N-terminus for coupling the fusion protein to a solid support. Suitable coupling elements are well known in the art and include, but are not limited to, cysteine residues, multiple lysine residues (such as 3-15 or 5-10) and multiple histidine residues (such as 3-15 or 5-10). The coupling element can be a cysteine residue located at the C-terminus of the fusion protein, allowing the fusion protein to be coupled to the solid support by reaction between the thiol group in the cysteine residue and the electrophilic group on the solid support. The coupling element can be directly attached to the polypeptide, alternatively, it can be attached to the N- or C-terminus of the polypeptide via a linker. The linker may be a linker commonly used in the art, and its presence will not affect the binding activity of the polypeptide. An exemplary linker is a linker sequence comprising or consisting of G and S, typically 2-20 amino acid residues in length. Exemplary linker sequences such as (GS)ₙ and (GSS)ₙ, etc, among which n can be an integer ranging from 2 to 10, while the total length of the linker usually will normally exceed 20 amino acid residues. The fusion protein may also comprise multiple amino acid residues (e.g., fewer than 15, 10 or 5) at the N-terminus, derived from the cloning process or residues from signal transduction sequences. As a specific example, the fusion protein may comprise AQ at its N-terminus.

In one or more embodiments, among the 2-8 polypeptides contained in the fusion protein of the present description, at least one polypeptide has a substitution mutation at position 15 as described in any of the embodiments described herein, and optionally has a substitution mutation at least at one position, at least at two positions, at least at three positions, or at all four positions selected from positions 3, 6, 9, and 23 as described in any of the embodiments described herein. Preferably, among the polypeptides contained in the fusion protein, at least one polypeptide has a substitution mutation described in any of the embodiments of the present description at positions 3, 9, and 15, and has a substitution mutation described in any of the embodiments of the present description at positions 6 and/or 23. Preferably, the substitution mutation at position 3 is N3L or N3V, the substitution mutation at position 6 is N6L or N6E, the substitution mutation at position 9 is Q91 or Q9F, and the substitution mutation at position 15 is E15L, E151 or E15V, the substitution mutation at position 23 is N23V, N231 or N23Y.

In some embodiments, the polypeptide of the present description that forms the fusion protein of the present description comprises at least one or more of the following polypeptides containing substitution mutation: SEQ ID NO: 4, SEQ ID NO: 8, SEQ ID NO: 9, SEQ ID NO: 10, SEQ ID NO: 11, SEQ ID NO: 14, SEQ ID NO: 16, SEQ ID NO: 18, SEQ ID NO: 21, SEQ ID NO: 22, SEQ ID NO: 51, SEQ ID NO: 53, SEQ ID NO: 60, SEQ ID NO: 71, EQ ID NO: 64, SEQ ID NO: 69, SEQ ID NO: 70, SEQ ID NO: 68, SEQ ID NO: 118, SEQ ID NO: 119, SEQ ID NO: 120, SEQ ID NO: 121, SEQ ID NO: 122, SEQ ID NO: 123 and SEQ ID NO: 124. The amino acid sequence of an exemplary fusion protein in the present description is shown in any one of SEQ ID NO: 73-109 and SEQ ID NO: 111-117.

The present description also provides a recombinant protein A, whose B domain is a Z domain mutant or a B domain mutant as described in any embodiment of the present description. As it is well-known in the field, the remaining domains of recombinant protein A can be the E domain, D domain, A domain, and C domain, comprising native E domain, D domain, A domain, and B domain, as well as known mutated E domain, D domain, A domain, and/or C domain that retain biological activity. Except for the B domain using the polypeptide of the present description, the known mutations or modifications to the rest domains of the protein A can all be used in the recombinant protein A of the present description.

The present description also comprises peptides, fusion proteins, or recombinant proteins A **having sequence having** have at least 85% identity with the peptides, fusion proteins, or recombinant protein A of any of the embodiments described herein, and having substitution mutations at one or more amino acid positions selected from positions 3, 6, 9, 15, and 23 corresponding to SEQ ID NO: 1 or 2 as described in any of the embodiments described herein. Herein, the "at least 85% identity" related to the amino acid sequence and the nucleotide sequence refers to an identity of more than 85%, preferably more than 90%, more preferably more than 95%, and even more preferably more than 97%, more preferably more than 98%, and still more preferably more than 99%. The identity between two aligned sequences can be calculated using tools well known in the art, including BLASTP for amino acid sequence alignment, and so on. In preferred embodiments, the present description comprises mutants of any amino acid sequence shown in SEQ ID NO: 3-71, 118-124 and 73-109, 111-117, each with at least 85%, preferably at least 90%, more preferably at least 97%, more preferably at least 99% identity compared to the corresponding parent sequence, and their amino acid residues at positions 3, 6, 9 and 15 of the corresponding parent sequence remain the same as the corresponding parent amino acid residues.

The present description also comprises the nucleic acid molecules encoding the aforementioned polypeptides, fusion proteins and recombinant protein A and the complementary sequences of the nucleic acid molecules. The present description comprises all forms of the nucleic acid molecules of the invention, comprising RNA and DNA. It should be understood that the complementary sequence described herein refers to a complementary sequence whose length is substantially the same as that of the nucleic acid molecule.

Nucleic acid constructs comprising the nucleic acid molecule or its complement are also covered by the scope of the present description. The nucleic acid construct may be an expression cassette comprising a promoter, the nucleic acid molecule and a transcription termination sequence. Among which, promoters and transcription termination sequences are well known in the art, and can be appropriately selected by those skilled in the art according to the host cell selected for expressing the polypeptide, fusion protein or recombinant protein A.

In some embodiments, nucleic acid constructs are vectors, comprising expression vectors and cloning vectors. Expression vectors are suitable for expressing exogenous genes in host cells, for example, nucleic acid molecules encoding polypeptides, fusion protein or recombinant protein A described in this description, comprising prokaryotic expression vectors and eukaryotic expression vectors. Eukaryotic expression systems comprise yeast expression systems, mammalian cell expression systems and insect cell expression systems. The cloning vector is used to amplify the target gene (such as the nucleic acid molecule encoding the polypeptide, fusion protein or protein A of the present description) in the host cell. Cloning vectors comprise plasmid vectors, phage vectors, viral vectors, as well as vectors combined with each other or another genomic DNA. The most commonly used host cell in molecular cloning is *Escherichia coli.*

The present description also provides an expression system comprising the nucleic acid molecule, nucleic acid construct or vector disclosed herein. The expression system can be, for example, a Gram-positive or Gram-negative prokaryotic host cell system, such as *E. coli* or *Bacillus* that has been modified to express the peptides, the fusion proteins, or the recombinant protein A of the present description. In some embodiments, the expression system is a eukaryotic host cell system, such as *yeast*, such as *Pichia pastoris* or *Saccharomyces cerevisiae.*

The present description also provides a separation medium . The separation matrices of the present description can be used to separate immunoglobulins or Fc-containing proteins. The separation medium of the present description comprises the polypeptide, fusion protein and/or recombinant protein A described in any embodiment of the present description coupled to a solid support. Due to the improved alkaline stability of the polypeptide, fusion protein and recombinant protein A of the present description, the separation medium of the present description can withstand highly alkaline conditions (0.5-2.0 M NaOH) during cleaning.

The solid support (matrix) in the separation medium of the present description may be a solid support (matrix) known in the art which used in the separation of immunoglobulin or Fc-containing protein.

Herein, solid phase support may be in any shape including particle, film, plate, tube, needle, and fiber. The solid support of the present description may be porous or non-porous material. In some embodiments, the solid support is in the form of porous or non-porous beads or particles. Matrix in beaded or granular form can be used as packed beds or suspension. Suspension includes expanded beds in which the particles or beads can move freely.

When the solid phase support is a particle, its particle size is preferably 20-200 µm.. For example, in case of solid support is a synthetic polymer, the particle size is preferably 20-100 µm, more preferably 30-80 µm. When the solid phase support is polysaccharide, the particle size is preferably 50-200 µm, more preferably 60-150 µm. The "particle size" in this specification refers to the average volume particle size measured by the ektacytometry based on ISO 13320 and JIS Z 8825-1.

Examples of solid supports include, but are not limited to, polymers containing polyhydroxyl groups, such as polysaccharides. Polysaccharides include dextran, starch, cellulose, pullulan, agar and agarose, etc. In preferred embodiments, the solid support comprises agar or agarose.

In some embodiments, the solid supports of the invention are synthetic polymers with hydrophilic surfaces. For example, such polymers can be synthetic polymers with hydroxyl, carboxyl, amino carbonyl, amino or oligoethylene oxide or polyethylene oxide groups on the outer surface (and on the inner surface if presence) through hydrophilic treatment, preferably synthetic polymer obtained by crosslinking multifunctional monomers such as methyl methacrylate and divinylbenzene. Exemplary synthetic polymers comprise polyvinyl alcohol, polystyrene, polystyrene divinylbenzene, polyhydroxyalkyl acrylates, polyhydroxyalkyl methacrylates, polyacrylamides, polymethacrylamides, and the like. In the case of polymer whose surface is hydrophobic, the surface can be hydrophilized to expose the hydrophilic groups to the surrounding aqueous liquid.

In some embodiments, the solid supports of the present description comprise supports of an inorganic nature, including, but not limited to, silica, zirconia, and the like.

In some embodiments, the solid support of the invention is in the form of a surface, chip, capillary or filter, eg, a membrane and so on.

In the present description, the polypeptide, fusion protein and recombinant protein A of the present description can be linked to the corresponding solid support through conventional coupling techniques through the thiol group, amino group and/or carboxyl group contained therein. Commonly used coupling reagents include, but are not limited to, diepoxides, epichlorohydrin, CNBr, and N-hydroxysuccinimide. A spacer can be introduced between the solid support and the polypeptide, fusion protein and recombinant protein A of the present description, which can facilitate their chemical coupling with the solid support.

In some embodiments, the polypeptide, fusion protein or recombinant protein A is coupled to the solid support through a thioether bond. In some embodiments, the polypeptide, fusion protein or recombinant protein A of the present description is coupled through its C-terminal cysteine, wherein the cysteine thiol group is effectively coupled to the electrophilic group (such as epoxy group, halohydrin group, etc.) on the solid support, forming a thioether bridge coupling.

In the separation medium of the present description, the concentration of the ligands coupled to the solid support (such as polypeptide, fusion protein and/or recombinant protein A as described in the present description) is usually 5-20 mg/ml, such as 5-15 mg/ml. The amount of the ligands coupled can be controlled by adjusting the concentration of the polypeptide, fusion protein and/or recombinant protein A used in the coupling process, the coupling conditions and/or the pore structure of the solid support.

The present description also provides a chromatographic column containing the separation medium of the present description. Usually, the separation medium of the present description is packed in the chromatographic column.

The present description also provides a method for separating Fc-containing protein (such as immunoglobulin), the method comprising a step where a sample containing Fc containing protein comes in contact with the peptide, the fusion protein, and/or the recombinant protein A described in any embodiment of the present description. In some embodiments, the method comprises a step where the sample are in contact with the separation medium or chromatography column described in any of the embodiments herein.

More specifically, in some embodiments, the method comprises:
(1) contacting the sample containing an Fc-containing protein with a separation medium according to any one of the embodiments herein;
(2) washing the separation medium ;
(3) eluting the Fc-containing protein from the separation medium;
(4) washing the separation medium ;

Herein, the samples can be various types of samples containing Fc-containing proteins (especially immunoglobulins). Preferably, the immunoglobulin referred to herein refers to IgG.

The washing buffer used in the method for washing the separation medium, the elution buffer for eluting the protein and the cleaning buffer for cleaning the separation medium are all commonly used washing buffers, elution buffers, and cleaning buffers in this field (especially in the field of protein A chromatography). For example, the washing buffer can be PBS solution. The elution buffer can be a solution or buffer with (pH≤5), preferably, the pH of the elution buffer is 2.5-5 or 3-5. In some embodiments, the pH of the elution buffer is 11 or higher, eg, the pH of the elution buffer is 11-14 or 11-13. In some embodiments, elution is performed using a citrate solution, such as a sodium citrate solution. Cleaning buffers are usually alkaline and can have a pH of 13-14. In some embodiments, the cleaning buffers is sodium hydroxide solution or potassium hydroxide solution with concentration of 0.1-2.0 M (such as 0.5-2.0 M or 0.5-1.0 M).

In some embodiments, the methods described herein further includes steps of collecting the eluate, and further separating and purifying the eluate.. According to the separated protein, further separation and purification can be carried out by anion or cation exchange chromatography, mixed-mode chromatography and/or hydrophobic interaction chromatography, etc.

The present description also provides the use of the polypeptide, fusion protein and protein A described in any embodiment herein in the separation and purification process of Fc-containing proteins, or in the preparation of separation medium for the separation and purification of Fc-containing proteins.

The sequence described in the present description is as follows:
SEQ ID NO: 1, (Native Native B domain)
SEQ ID NO: 2, (Z domain)
SEQ ID NO: 3, Z(N3L) 
VDLKFNKEQQNAFYEILHLPNLNEEQRNAFIQSLKDDPSQSANLLAEA
KKLNDAQAPK
SEQ ID NO: 4, Z(N3V) VDVKFNKEQQNAFYEILHLPNLNEEQRNAFIQSLKDDPSQSANLLAEA KKLNDAQAPK
SEQ ID NO: 5, Z(N3Y) 
VDYKFNKEQQNAFYEILHLPNLNEEQRNAFIQSLKDDPSQSANLLAEA
KKLNDAQAPK
SEQ ID NO: 6, Z(N6S) 
VDNKFSKEQQNAFYEILHLPNLNEEQRNAFIQSLKDDPSQSANLLAEA
KKLNDAQAPK
SEQ ID NO: 7, Z(N6L) 
VDNKFLKEQQNAFYEILHLPNLNEEQRNAFIQSLKDDPSQSANLLAEA
KKLNDAQAPK
SEQ ID NO: 8, Z(N6E) 
VDNKFEKEQQNAFYEILHLPNLNEEQRNAFIQSLKDDPSQSANLLAEA
KKLNDAQAPK
SEQ ID NO: 9, Z(Q9I) 
VDNKFNKEIQNAFYEILHLPNLNEEQRNAFIQSLKDDPSQSANLLAEAK
KLNDAQAPK
SEQ ID NO: 10, Z(Q9F) 
VDNKFNKEFQNAFYEILHLPNLNEEQRNAFIQSLKDDPSQSANLLAEA
KKLNDAQAPK
SEQ ID NO: 11, Z(Q9M) 
VDNKFNKEMQNAFYEILHLPNLNEEQRNAFIQSLKDDPSQSANLLAEA
KKLNDAQAPK
SEQ ID NO: 12, Z(E15T) 
VDNKFNKEQQNAFYTILHLPNLNEEQRNAFIQSLKDDPSQSANLLAEA
KKLNDAQAPK
SEQ ID NO: 13, Z(E15W) 
VDNKFNKEQQNAFYWILHLPNLNEEQRNAFIQSLKDDPSQSANLLAEA
KKLNDAQAPK
SEQ ID NO: 14, Z(E15L) 
VDNKFNKEQQNAFYLILHLPNLNEEQRNAFIQSLKDDPSQSANLLAEA
KKLNDAQAPK
SEQ ID NO: 15, Z(E15V) 
VDNKFNKEQQNAFYVILHLPNLNEEQRNAFIQSLKDDPSQSANLLAEA
KKLNDAQAPK
SEQ ID NO: 16, Z(E15I) 
VDNKFNKEQQNAFYIILHLPNLNEEQRNAFIQSLKDDPSQSANLLAEA
KKLNDAQAPK
SEQ ID NO: 17, Z(E15F) 
VDNKFNKEQQNAFYFILHLPNLNEEQRNAFIQSLKDDPSQSANLLAEA
KKLNDAQAPK
SEQ ID NO: 18, Z(E15S) 
VDNKFNKEQQNAFYSILHLPNLNEEQRNAFIQSLKDDPSQSANLLAEA
KKLNDAQAPK
SEQ ID NO: 19, Z(E15Y) 
VDNKFNKEQQNAFYYILHLPNLNEEQRNAFIQSLKDDPSQSANLLAEA
KKLNDAQAPK
SEQ ID NO: 20, Z(E15D) 
VDNKFNKEQQNAFYDILHLPNLNEEQRNAFIQSLKDDPSQSANLLAEA
KKLNDAQAPK
SEQ ID NO: 21, Z(N23V) 
VDNKFNKEQQNAFYEILHLPNLVEEQRNAFIQSLKDDPSQSANLLAEA
KKLNDAQAPK
SEQ ID NO: 22, Z(N23I) 
VDNKFNKEQQNAFYEILHLPNLIEEQRNAFIQSLKDDPSQSANLLAEAK
KLNDAQAPK
SEQ ID NO: 23, Z(N23Y) 
VDNKFNKEQQNAFYEILHLPNLYEEQRNAFIQSLKDDPSQSANLLAEA
KKLNDAQAPK
SEQ ID NO: 24, Z(Q9I,E15T) 
VDNKFNKEIQNAFYTILHLPNLNEEQRNAFIQSLKDDPSQSANLLAEAK
KLNDAQAPK
SEQ ID NO: 25, Z(Q91,E15W) 
VDNKFNKEIQNAFYWILHLPNLNEEQRNAFIQSLKDDPSQSANLLAEA
KKLNDAQAPK
SEQ ID NO: 26, Z(Q9I,E15L) 
VDNKFNKEIQNAFYLILHLPNLNEEQRNAFIQSLKDDPSQSANLLAEAK
KLNDAQAPK
SEQ ID NO: 27, Z(Q9I,E15V) 
VDNKFNKEIQNAFYVILHLPNLNEEQRNAFIQSLKDDPSQSANLLAEA
KKLNDAQAPK
SEQ ID NO: 28, Z(Q9I,E15I) 
VDNKFNKEIQNAFYIILHLPNLNEEQRNAFIQSLKDDPSQSANLLAEAK
KLNDAQAPK
SEQ ID NO: 29, Z(Q9I,E15F) 
VDNKFNKEIQNAFYFILHLPNLNEEQRNAFIQSLKDDPSQSANLLAEAK
KLNDAQAPK
SEQ ID NO: 30, Z(Q9I,E15S) 
VDNKFNKEIQNAFYSILHLPNLNEEQRNAFIQSLKDDPSQSANLLAEAK
KLNDAQAPK
SEQ ID NO: 31, Z(Q9I,E15Y) 
VDNKFNKEIQNAFYYILHLPNLNEEQRNAFIQSLKDDPSQSANLLAEA
KKLNDAQAPK
SEQ ID NO: 32, Z(Q9I,E15D) VDNKFNKEIQNAFYDILHLPNLNEEQRNAFIQSLKDDPSQSANLLAEA KKLNDAQAPK
SEQ ID NO: 33, Z(Q9F,E15T) 
VDNKFNKEFQNAFYTILHLPNLNEEQRNAFIQSLKDDPSQSANLLAEA
KKLNDAQAPK
SEQ ID NO: 34, Z(Q9F,E15W) 
VDNKFNKEFQNAFYWILHLPNLNEEQRNAFIQSLKDDPSQSANLLAEA
KKLNDAQAPK
SEQ ID NO: 35, Z(Q9F,E15L) 
VDNKFNKEFQNAFYLILHLPNLNEEQRNAFIQSLKDDPSQSANLLAEA
KKLNDAQAPK
SEQ ID NO: 36, Z(Q9F,E15V) 
VDNKFNKEFQNAFYVILHLPNLNEEQRNAFIQSLKDDPSQSANLLAEA
KKLNDAQAPK
SEQ ID NO: 37, Z(Q9F,E15I) 
VDNKFNKEFQNAFYIILHLPNLNEEQRNAFIQSLKDDPSQSANLLAEAK
KLNDAQAPK
SEQ ID NO: 38, Z(Q9F,E15F) 
VDNKFNKEFQNAFYFILHLPNLNEEQRNAFIQSLKDDPSQSANLLAEA
KKLNDAQAPK
SEQ ID NO: 39, Z(Q9F,E15S) 
VDNKFNKEFQNAFYSILHLPNLNEEQRNAFIQSLKDDPSQSANLLAEA
KKLNDAQAPK
SEQ ID NO: 40, Z(Q9F,E15Y) 
VDNKFNKEFQNAFYYILHLPNLNEEQRNAFIQSLKDDPSQSANLLAEA
KKLNDAQAPK
SEQ ID NO: 41, Z(Q9F,E15D) 
VDNKFNKEFQNAFYDILHLPNLNEEQRNAFIQSLKDDPSQSANLLAEA
KKLNDAQAPK
SEQ ID NO: 42, Z(N3L,Q9I,E15L) 
VDLKFNKEIQNAFYLILHLPNLNEEQRNAFIQSLKDDPSQSANLLAEAK
KLNDAQAPK
SEQ ID NO: 43, Z(N3Y,Q9I,E15V) 
VDYKFNKEIQNAFYVILHLPNLNEEQRNAFIQSLKDDPSQSANLLAEA
KKLNDAQAPK
SEQ ID NO: 44, Z(N3V,Q9I,E15I) 
VDVKFNKEIQNAFYIILHLPNLNEEQRNAFIQSLKDDPSQSANLLAEAK
KLNDAQAPK
SEQ ID NO: 45, Z(N6E,Q9I,E15L) 
VDNKFEKEIQNAFYLILHLPNLNEEQRNAFIQSLKDDPSQSANLLAEAK
KLNDAQAPK
SEQ ID NO: 46, Z(N6E,Q9F,E15V) 
VDNKFEKEFQNAFYVILHLPNLNEEQRNAFIQSLKDDPSQSANLLAEA
KKLNDAQAPK
SEQ ID NO: 47, Z(N6E, Q9I,E15I) 
VDNKFEKEIQNAFYIILHLPNLNEEQRNAFIQSLKDDPSQSANLLAEAK
KLNDAQAPK
SEQ ID NO: 48, Z(Q9I,E15L,N23V) 
VDVKFNKEIQNAFYLILHLPNLNEEQRNAFIQSLKDDPSQSANLLAEAK
KLNDAQAPK
SEQ ID NO: 49, Z(Q9I,E15V,N23V) 
VDVKFNKEIQNAFYVILHLPNLNEEQRNAFIQSLKDDPSQSANLLAEA
KKLNDAQAPK
SEQ ID NO: 50, Z(Q9I,E15I,N23V) 
VDVKFNKEIQNAFYIILHLPNLNEEQRNAFIQSLKDDPSQSANLLAEAK
KLNDAQAPK
SEQ ID NO: 51, Z(N3L,N6L,Q9I,E15L) 
VDLKFLKEIQNAFYLILHLPNLNEEQRNAFIQSLKDDPSQSANLLAEAK
KLNDAQAPK
SEQ ID NO: 52, Z(N3Y,N6E,Q9I,E15I) 
VDYKFEKEIQNAFYIILHLPNLNEEQRNAFIQSLKDDPSQSANLLAEAK
KLNDAQAPK
SEQ ID NO: 53, Z(N3V,N6E,Q9I,E15L) 
VDVKFEKEIQNAFYLILHLPNLNEEQRNAFIQSLKDDPSQSANLLAEAK
KLNDAQAPK
SEQ ID NO: 54, Z(N3L,N6L,Q9F,E15I) 
VDLKFLKEFQNAFYIILHLPNLNEEQRNAFIQSLKDDPSQSANLLAEAK
KLNDAQAPK
SEQ ID NO: 55, Z(N3Y,N6E,Q9I,E15L) 
VDYKFEKEIQNAFYLILHLPNLNEEQRNAFIQSLKDDPSQSANLLAEAK
KLNDAQAPK
SEQ ID NO: 56, Z(N3V,N6E,Q9I,E15I) 
VDVKFEKEIQNAFYIILHLPNLNEEQRNAFIQSLKDDPSQSANLLAEAK
KLNDAQAPK
SEQ ID NO: 57, Z(N3L,Q9I,E15L,N23V) 
VDLKFNKEIQNAFYLILHLPNLVEEQRNAFIQSLKDDPSQSANLLAEAK
KLNDAQAPK
SEQ ID NO: 58, Z(N3Y,Q9I,E15I,N23I) 
VDYKFNKEIQNAFYIILHLPNLVEEQRNAFIQSLKDDPSQSANLLAEAK
KLNDAQAPK
SEQ ID NO: 59, Z(N3V,Q9I,E15L,N23Y) 
VDVKFNKEIQNAFYLILHLPNLYEEQRNAFIQSLKDDPSQSANLLAEAK
KLNDAQAPK
SEQ ID NO: 60, Z(N6L,Q9F,E15I,N23V) VDNKFLKEFQNAFYIILHLPNLVEEQRNAFIQSLKDDPSQSANLLAEAK KLNDAQAPK
SEQ ID NO: 61, Z(N6E,Q9I,E15L,N23I) 
VDNKFEKEIQNAFYLILHLPNLIEEQRNAFIQSLKDDPSQSANLLAEAK
KLNDAQAPK
SEQ ID NO: 62, Z(N6E,Q9I,E15L,N23Y) 
VDNKFEKEIQNAFYLILHLPNLYEEQRNAFIQSLKDDPSQSANLLAEAK
KLNDAQAPK
SEQ ID NO: 63, Z(N3L,N6L,Q9I,E15L,N23V) 
VDLKFLKEIQNAFYLILHLPNLVEEQRNAFIQSLKDDPSQSANLLAEAK
KLNDAQAPK
SEQ ID NO: 64, Z(N3L,N6E,Q9I,E15I,N23I) 
VDLKFEKEIQNAFYIILHLPNLIEEQRNAFIQSLKDDPSQSANLLAEAKK
LNDAQAPK
SEQ ID NO: 65, Z(N3V,N6E,Q9I,E15L,N23Y) 
VDVKFEKEIQNAFYLILHLPNLYEEQRNAFIQSLKDDPSQSANLLAEAK
KLNDAQAPK
SEQ ID NO: 66, Z(N3V,N6E,Q9I,E15I,N23Y) 
VDVKFEKEIQNAFYIILHLPNLYEEQRNAFIQSLKDDPSQSANLLAEAK
KLNDAQAPK
SEQ ID NO: 67, Z(N3Y,N6E,Q9I,E15L,N23Y) 
VDYKFEKEIQNAFYLILHLPNLYEEQRNAFIQSLKDDPSQSANLLAEAK
KLNDAQAPK
SEQ ID NO: 68, Z(N3V,N6L,Q9I,E15V,N23Y) 
VDVKFLKEIQNAFYVILHLPNLYEEQRNAFIQSLKDDPSQSANLLAEAK
KLNDAQAPK
SEQ ID NO: 69, Z(N3L,N6L,Q9F,E15I,N23V) 
VDLKFLKEFQNAFYIILHLPNLVEEQRNAFIQSLKDDPSQSANLLAEAK
KLNDAQAPK
SEQ ID NO: 70, Z(N3L,N6E,Q9F,E15L,N23I) 
VDLKFEKEFQNAFYLILHLPNLIEEQRNAFIQSLKDDPSQSANLLAEAK
KLNDAQAPK
SEQ ID NO: 71, Z(N3V,N6L,Q9F,E15V,N23Y) 
VDVKFLKEFQNAFYVILHLPNLYEEQRNAFIQSLKDDPSQSANLLAEA
KKLNDAQAPK
SEQ ID NO: 72, Z(Z domain)4 
VDNKFNKEQQNAFYEILHLPNLNEEQRNAFIQSLKDDPSQSANLLAEA
KKLNDAQAPKVDNKFNKEQQNAFYEILHLPNLNEEQRNAFIQSLKDDPSQS
ANLLAEAKKLNDAQAPKVDNKFNKEQQNAFYEILHLPNLNEEQRNAFIQSL
KDDPSQSANLLAEAKKLNDAQAPKVDNKFNKEQQNAFYEILHLPNLNEEQ
RNAFIQSLKDDPSQSANLLAEAKKLNDAQAPK
SEQ ID NO: 73, Z(N3L)4 
VDLKFNKEQQNAFYEILHLPNLNEEQRNAFIQSLKDDPSQSANLLAEA
KKLNDAQAPKVDLKFNKEQQNAFYEILHLPNLNEEQRNAFIQSLKDDPSQS
ANLLAEAKKLNDAQAPKVDLKFNKEQQNAFYEILHLPNLNEEQRNAFIQSL
KDDPSQSANLLAEAKKLNDAQAPKVDLKFNKEQQNAFYEILHLPNLNEEQ
RNAFIQSLKDDPSQSANLLAEAKKLNDAQAPK
SEQ ID NO: 74, Z(N3V)4 
VDVKFNKEQQNAFYEILHLPNLNEEQRNAFIQSLKDDPSQSANLLAEA
KKLNDAQAPKVDVKFNKEQQNAFYEILHLPNLNEEQRNAFIQSLKDDPSQS
ANLLAEAKKLNDAQAPKVDVKFNKEQQNAFYEILHLPNLNEEQRNAFIQSL
KDDPSQSANLLAEAKKLNDAQAPKVDVKFNKEQQNAFYEILHLPNLNEEQ
RNAFIQSLKDDPSQSANLLAEAKKLNDAQAPK
SEQ ID NO: 75, Z(N6L)4 
VDNKFLKEQQNAFYEILHLPNLNEEQRNAFIQSLKDDPSQSANLLAEA
KKLNDAQAPKVDNKFLKEQQNAFYEILHLPNLNEEQRNAFIQSLKDDPSQS
ANLLAEAKKLNDAQAPKVDNKFLKEQQNAFYEILHLPNLNEEQRNAFIQSL
 
KDDPSQSANLLAEAKKLNDAQAPKVDNKFLKEQQNAFYEILHLPNLNEEQ
RNAFIQSLKDDPSQSANLLAEAKKLNDAQAPK
SEQ ID NO: 76, Z(N6E)4 
VDNKFEKEQQNAFYEILHLPNLNEEQRNAFIQSLKDDPSQSANLLAEA
KKLNDAQAPKVDNKFEKEQQNAFYEILHLPNLNEEQRNAFIQSLKDDPSQS
ANLLAEAKKLNDAQAPKVDNKFEKEQQNAFYEILHLPNLNEEQRNAFIQSL
KDDPSQSANLLAEAKKLNDAQAPKVDNKFEKEQQNAFYEILHLPNLNEEQ
RNAFIQSLKDDPSQSANLLAEAKKLNDAQAPK
SEQ ID NO: 77, Z(Q9I)4 
VDNKFNKEIQNAFYEILHLPNLNEEQRNAFIQSLKDDPSQSANLLAEAK
KLNDAQAPKVDNKFNKEIQNAFYEILHLPNLNEEQRNAFIQSLKDDPSQSA
NLLAEAKKLNDAQAPKVDNKFNKEIQNAFYEILHLPNLNEEQRNAFIQSLK
DDPSQSANLLAEAKKLNDAQAPKVDNKFNKEIQNAFYEILHLPNLNEEQRN
AFIQSLKDDPSQSANLLAEAKKLNDAQAPK
SEQ ID NO: 78, Z(Q9F)4 
VDNKFNKEFQNAFYEILHLPNLNEEQRNAFIQSLKDDPSQSANLLAEA
KKLNDAQAPKVDNKFNKEFQNAFYEILHLPNLNEEQRNAFIQSLKDDPSQS
ANLLAEAKKLNDAQAPKVDNKFNKEFQNAFYEILHLPNLNEEQRNAFIQSL
KDDPSQSANLLAEAKKLNDAQAPKVDNKFNKEFQNAFYEILHLPNLNEEQ
RNAFIQSLKDDPSQSANLLAEAKKLNDAQAPK
SEQ ID NO: 79, Z(Q9M)4 
VDNKFNKEMQNAFYEILHLPNLNEEQRNAFIQSLKDDPSQSANLLAEA
KKLNDAQAPKVDNKFNKEMQNAFYEILHLPNLNEEQRNAFIQSLKDDPSQS
ANLLAEAKKLNDAQAPKVDNKFNKEMQNAFYEILHLPNLNEEQRNAFIQS
LKDDPSQSANLLAEAKKLNDAQAPKVDNKFNKEMQNAFYEILHLPNLNEE
QRNAFIQSLKDDPSQSANLLAEAKKLNDAQAPK
SEQ ID NO: 80, Z(E15T)4 VDNKFNKEQQNAFYTILHLPNLNEEQRNAFIQSLKDDPSQSANLLAEA 
KKLNDAQAPKVDNKFNKEQQNAFYTILHLPNLNEEQRNAFIQSLKDDPSQS
ANLLAEAKKLNDAQAPKVDNKFNKEQQNAFYTILHLPNLNEEQRNAFIQSL
KDDPSQSANLLAEAKKLNDAQAPKVDNKFNKEQQNAFYTILHLPNLNEEQ
RNAFIQSLKDDPSQSANLLAEAKKLNDAQAPK
SEQ ID NO: 81, Z(E15W)4 
VDNKFNKEQQNAFYWILHLPNLNEEQRNAFIQSLKDDPSQSANLLAEA
KKLNDAQAPKVDNKFNKEQQNAFYWILHLPNLNEEQRNAFIQSLKDDPSQ
SANLLAEAKKLNDAQAPKVDNKFNKEQQNAFYWILHLPNLNEEQRNAFIQ
SLKDDPSQSANLLAEAKKLNDAQAPKVDNKFNKEQQNAFYWILHLPNLNE
EQRNAFIQSLKDDPSQSANLLAEAKKLNDAQAPK
SEQ ID NO: 82, Z(E15L)4 
VDNKFNKEQQNAFYLILHLPNLNEEQRNAFIQSLKDDPSQSANLLAEA
KKLNDAQAPKVDNKFNKEQQNAFYLILHLPNLNEEQRNAFIQSLKDDPSQS
ANLLAEAKKLNDAQAPKVDNKFNKEQQNAFYLILHLPNLNEEQRNAFIQSL
KDDPSQSANLLAEAKKLNDAQAPKVDNKFNKEQQNAFYLILHLPNLNEEQ
RNAFIQSLKDDPSQSANLLAEAKKLNDAQAPK
SEQ ID NO: 83, Z(E15V)4 
VDNKFNKEQQNAFYVILHLPNLNEEQRNAFIQSLKDDPSQSANLLAEA
KKLNDAQAPKVDNKFNKEQQNAFYVILHLPNLNEEQRNAFIQSLKDDPSQS
ANLLAEAKKLNDAQAPKVDNKFNKEQQNAFYVILHLPNLNEEQRNAFIQSL
KDDPSQSANLLAEAKKLNDAQAPKVDNKFNKEQQNAFYVILHLPNLNEEQ
RNAFIQSLKDDPSQSANLLAEAKKLNDAQAPK
SEQ ID NO: 84, Z(E151)4 
VDNKFNKEQQNAFYIILHLPNLNEEQRNAFIQSLKDDPSQSANLLAEA
KKLNDAQAPKVDNKFNKEQQNAFYIILHLPNLNEEQRNAFIQSLKDDPSQS
ANLLAEAKKLNDAQAPKVDNKFNKEQQNAFYIILHLPNLNEEQRNAFIQSL
KDDPSQSANLLAEAKKLNDAQAPKVDNKFNKEQQNAFYIILHLPNLNEEQR
NAFIQSLKDDPSQSANLLAEAKKLNDAQAPK
SEQ ID NO: 85, Z(E15F)4 
VDNKFNKEQQNAFYFILHLPNLNEEQRNAFIQSLKDDPSQSANLLAEA
KKLNDAQAPKVDNKFNKEQQNAFYFILHLPNLNEEQRNAFIQSLKDDPSQS
ANLLAEAKKLNDAQAPKVDNKFNKEQQNAFYFILHLPNLNEEQRNAFIQSL
KDDPSQSANLLAEAKKLNDAQAPKVDNKFNKEQQNAFYFILHLPNLNEEQ
RNAFIQSLKDDPSQSANLLAEAKKLNDAQAPK
SEQ ID NO: 86, Z(E15S)4 
VDNKFNKEQQNAFYSILHLPNLNEEQRNAFIQSLKDDPSQSANLLAEA
KKLNDAQAPKVDNKFNKEQQNAFYSILHLPNLNEEQRNAFIQSLKDDPSQS
ANLLAEAKKLNDAQAPKVDNKFNKEQQNAFYSILHLPNLNEEQRNAFIQSL
KDDPSQSANLLAEAKKLNDAQAPKVDNKFNKEQQNAFYSILHLPNLNEEQ
RNAFIQSLKDDPSQSANLLAEAKKLNDAQAPK
SEQ ID NO: 87, Z(E15Y)4 
VDNKFNKEQQNAFYYILHLPNLNEEQRNAFIQSLKDDPSQSANLLAEA
KKLNDAQAPKVDNKFNKEQQNAFYYILHLPNLNEEQRNAFIQSLKDDPSQS
ANLLAEAKKLNDAQAPKVDNKFNKEQQNAFYYILHLPNLNEEQRNAFIQSL
KDDPSQSANLLAEAKKLNDAQAPKVDNKFNKEQQNAFYYILHLPNLNEEQ
RNAFIQSLKDDPSQSANLLAEAKKLNDAQAPK
SEQ ID NO: 88, Z(E15D)4 
VDNKFNKEQQNAFYDILHLPNLNEEQRNAFIQSLKDDPSQSANLLAEA
KKLNDAQAPKVDNKFNKEQQNAFYDILHLPNLNEEQRNAFIQSLKDDPSQS
ANLLAEAKKLNDAQAPKVDNKFNKEQQNAFYDILHLPNLNEEQRNAFIQSL
KDDPSQSANLLAEAKKLNDAQAPKVDNKFNKEQQNAFYDILHLPNLNEEQ
RNAFIQSLKDDPSQSANLLAEAKKLNDAQAPK
SEQ ID NO: 89, Z(N23V)4 
VDNKFNKEQQNAFYEILHLPNLVEEQRNAFIQSLKDDPSQSANLLAEA
KKLNDAQAPKVDNKFNKEQQNAFYEILHLPNLVEEQRNAFIQSLKDDPSQS
ANLLAEAKKLNDAQAPKVDNKFNKEQQNAFYEILHLPNLVEEQRNAFIQSL
 
KDDPSQSANLLAEAKKLNDAQAPKVDNKFNKEQQNAFYEILHLPNLVEEQ
RNAFIQSLKDDPSQSANLLAEAKKLNDAQAPK
SEQ ID NO: 90, Z(N231)4 
VDNKFNKEQQNAFYEILHLPNLIEEQRNAFIQSLKDDPSQSANLLAEAK
KLNDAQAPKVDNKFNKEQQNAFYEILHLPNLIEEQRNAFIQSLKDDPSQSA
NLLAEAKKLNDAQAPKVDNKFNKEQQNAFYEILHLPNLIEEQRNAFIQSLK
DDPSQSANLLAEAKKLNDAQAPKVDNKFNKEQQNAFYEILHLPNLIEEQRN
AFIQSLKDDPSQSANLLAEAKKLNDAQAPK
SEQ ID NO: 91, Z(N23Y)4 
VDNKFNKEQQNAFYEILHLPNLYEEQRNAFIQSLKDDPSQSANLLAEA
KKLNDAQAPKVDNKFNKEQQNAFYEILHLPNLYEEQRNAFIQSLKDDPSQS
ANLLAEAKKLNDAQAPKVDNKFNKEQQNAFYEILHLPNLYEEQRNAFIQSL
KDDPSQSANLLAEAKKLNDAQAPKVDNKFNKEQQNAFYEILHLPNLYEEQ
RNAFIQSLKDDPSQSANLLAEAKKLNDAQAPK
SEQ ID NO: 92, Z(Q9I,E15L)4 
VDNKFNKEIQNAFYLILHLPNLNEEQRNAFIQSLKDDPSQSANLLAEAK
KLNDAQAPKVDNKFNKEIQNAFYLILHLPNLNEEQRNAFIQSLKDDPSQSA
NLLAEAKKLNDAQAPKVDNKFNKEIQNAFYLILHLPNLNEEQRNAFIQSLK
DDPSQSANLLAEAKKLNDAQAPKVDNKFNKEIQNAFYLILHLPNLNEEQRN
AFIQSLKDDPSQSANLLAEAKKLNDAQAPK
SEQ ID NO: 93, Z(Q9I,E15V)4 
VDNKFNKEIQNAFYVILHLPNLNEEQRNAFIQSLKDDPSQSANLLAEA
KKLNDAQAPKVDNKFNKEIQNAFYVILHLPNLNEEQRNAFIQSLKDDPSQS
ANLLAEAKKLNDAQAPKVDNKFNKEIQNAFYVILHLPNLNEEQRNAFIQSL
KDDPSQSANLLAEAKKLNDAQAPKVDNKFNKEIQNAFYVILHLPNLNEEQR
NAFIQSLKDDPSQSANLLAEAKKLNDAQAPK
SEQ ID NO: 94, Z(Q9I,E15I)4 VDNKFNKEIQNAFYIILHLPNLNEEQRNAFIQSLKDDPSQSANLLAEAK 
KLNDAQAPKVDNKFNKEIQNAFYIILHLPNLNEEQRNAFIQSLKDDPSQSAN
LLAEAKKLNDAQAPKVDNKFNKEIQNAFYIILHLPNLNEEQRNAFIQSLKDD
PSQSANLLAEAKKLNDAQAPKVDNKFNKEIQNAFYIILHLPNLNEEQRNAFI
QSLKDDPSQSANLLAEAKKLNDAQAPK
SEQ ID NO: 95, Z(N3L,N6L,Q9I,E15L)4 
VDLKFLKEIQNAFYLILHLPNLNEEQRNAFIQSLKDDPSQSANLLAEAK
KLNDAQAPKVDLKFLKEIQNAFYLILHLPNLNEEQRNAFIQSLKDDPSQSAN
LLAEAKKLNDAQAPKVDLKFLKEIQNAFYLILHLPNLNEEQRNAFIQSLKDD
PSQSANLLAEAKKLNDAQAPKVDLKFLKEIQNAFYLILHLPNLNEEQRNAFI
QSLKDDPSQSANLLAEAKKLNDAQAPK
SEQ ID NO: 96, Z(N3Y,N6E,Q9I,E15I)4 
VDYKFEKEIQNAFYIILHLPNLNEEQRNAFIQSLKDDPSQSANLLAEAK
KLNDAQAPKVDYKFEKEIQNAFYIILHLPNLNEEQRNAFIQSLKDDPSQSAN
LLAEAKKLNDAQAPKVDYKFEKEIQNAFYIILHLPNLNEEQRNAFIQSLKDD
PSQSANLLAEAKKLNDAQAPKVDYKFEKEIQNAFYIILHLPNLNEEQRNAFI
QSLKDDPSQSANLLAEAKKLNDAQAPK
SEQ ID NO: 97, Z(N3V,N6E,Q9I,E15L)4 
VDVKFEKEIQNAFYLILHLPNLNEEQRNAFIQSLKDDPSQSANLLAEAK
KLNDAQAPKVDVKFEKEIQNAFYLILHLPNLNEEQRNAFIQSLKDDPSQSAN
LLAEAKKLNDAQAPKVDVKFEKEIQNAFYLILHLPNLNEEQRNAFIQSLKD
DPSQSANLLAEAKKLNDAQAPKVDVKFEKEIQNAFYLILHLPNLNEEQRNA
FIQSLKDDPSQSANLLAEAKKLNDAQAPK
SEQ ID NO: 98, Z(N3L,Q91,E15L,N23V)4 
VDLKFNKEIQNAFYLILHLPNLVEEQRNAFIQSLKDDPSQSANLLAEAK
KLNDAQAPKVDLKFNKEIQNAFYLILHLPNLVEEQRNAFIQSLKDDPSQSAN
LLAEAKKLNDAQAPKVDLKFNKEIQNAFYLILHLPNLVEEQRNAFIQSLKD
DPSQSANLLAEAKKLNDAQAPKVDLKFNKEIQNAFYLILHLPNLVEEQRNA
FIQSLKDDPSQSANLLAEAKKLNDAQAPK
SEQ ID NO: 99, Z(N6L,Q9F,E151,N23V)4 
VDNKFLKEFQNAFYIILHLPNLVEEQRNAFIQSLKDDPSQSANLLAEAK
KLNDAQAPKVDNKFLKEFQNAFYIILHLPNLVEEQRNAFIQSLKDDPSQSAN
LLAEAKKLNDAQAPKVDNKFLKEFQNAFYIILHLPNLVEEQRNAFIQSLKD
DPSQSANLLAEAKKLNDAQAPKVDNKFLKEFQNAFYIILHLPNLVEEQRNA
FIQSLKDDPSQSANLLAEAKKLNDAQAPK
SEQ ID NO: 100, Z(N3L,N6L,Q9I,E15L,N23V)4 
VDLKFLKEIQNAFYLILHLPNLVEEQRNAFIQSLKDDPSQSANLLAEAK
KLNDAQAPKVDLKFLKEIQNAFYLILHLPNLVEEQRNAFIQSLKDDPSQSAN
LLAEAKKLNDAQAPKVDLKFLKEIQNAFYLILHLPNLVEEQRNAFIQSLKDD
PSQSANLLAEAKKLNDAQAPKVDLKFLKEIQNAFYLILHLPNLVEEQRNAFI
QSLKDDPSQSANLLAEAKKLNDAQAPK
SEQ ID NO: 101, Z(N3L,N6E,Q9I,E15I,N23I)4 
VDLKFEKEIQNAFYIILHLPNLIEEQRNAFIQSLKDDPSQSANLLAEAKK
LNDAQAPKVDLKFEKEIQNAFYIILHLPNLIEEQRNAFIQSLKDDPSQSANLL
AEAKKLNDAQAPKVDLKFEKEIQNAFYIILHLPNLIEEQRNAFIQSLKDDPSQ
SANLLAEAKKLNDAQAPKVDLKFEKEIQNAFYIILHLPNLIEEQRNAFIQSLK
DDPSQSANLLAEAKKLNDAQAPK
SEQ ID NO: 102, Z(N3V,N6L,Q9F,E15V,N23Y)4 
VDVKFLKEFQNAFYVILHLPNLYEEQRNAFIQSLKDDPSQSANLLAEA
KKLNDAQAPKVDVKFLKEFQNAFYVILHLPNLYEEQRNAFIQSLKDDPSQS
ANLLAEAKKLNDAQAPKVDVKFLKEFQNAFYVILHLPNLYEEQRNAFIQSL
KDDPSQSANLLAEAKKLNDAQAPKVDVKFLKEFQNAFYVILHLPNLYEEQ
RNAFIQSLKDDPSQSANLLAEAKKLNDAQAPK
SEQ ID NO: 103, Z(N3V,N6E,Q9I,E15I,N23Y)4 
VDVKFEKEIQNAFYIILHLPNLYEEQRNAFIQSLKDDPSQSANLLAEAK
KLNDAQAPKVDVKFEKEIQNAFYIILHLPNLYEEQRNAFIQSLKDDPSQSAN
LLAEAKKLNDAQAPKVDVKFEKEIQNAFYIILHLPNLYEEQRNAFIQSLKDD
 
PSQSANLLAEAKKLNDAQAPKVDVKFEKEIQNAFYIILHLPNLYEEQRNAFI
QSLKDDPSQSANLLAEAKKLNDAQAPK
SEQ ID NO: 104, Z(N3L,N6L,Q9F,E151,N23V)4 
VDLKFLKEFQNAFYIILHLPNLVEEQRNAFIQSLKDDPSQSANLLAEAK
KLNDAQAPKVDLKFLKEFQNAFYIILHLPNLVEEQRNAFIQSLKDDPSQSAN
LLAEAKKLNDAQAPKVDLKFLKEFQNAFYIILHLPNLVEEQRNAFIQSLKDD
PSQSANLLAEAKKLNDAQAPKVDLKFLKEFQNAFYIILHLPNLVEEQRNAFI
QSLKDDPSQSANLLAEAKKLNDAQAPK
SEQ ID NO: 105, Z(N3L,N6E,Q9F,E15L,N23I)4 
VDLKFEKEFQNAFYLILHLPNLIEEQRNAFIQSLKDDPSQSANLLAEAK
KLNDAQAPKVDLKFEKEFQNAFYLILHLPNLIEEQRNAFIQSLKDDPSQSAN
LLAEAKKLNDAQAPKVDLKFEKEFQNAFYLILHLPNLIEEQRNAFIQSLKDD
PSQSANLLAEAKKLNDAQAPKVDLKFEKEFQNAFYLILHLPNLIEEQRNAFI
QSLKDDPSQSANLLAEAKKLNDAQAPK
SEQ ID NO: 106, Z(N3Y,N6E,Q9I,E15L,N23Y)4 
VDYKFEKEIQNAFYLILHLPNLYEEQRNAFIQSLKDDPSQSANLLAEAK
KLNDAQAPKVDYKFEKEIQNAFYLILHLPNLYEEQRNAFIQSLKDDPSQSAN
LLAEAKKLNDAQAPKVDYKFEKEIQNAFYLILHLPNLYEEQRNAFIQSLKD
DPSQSANLLAEAKKLNDAQAPKVDYKFEKEIQNAFYLILHLPNLYEEQRNA
FIQSLKDDPSQSANLLAEAKKLNDAQAPK
SEQ ID NO: 107, Z(N3L,N6E,Q9I,E15I,N23I)6 
VDLKFEKEIQNAFYIILHLPNLIEEQRNAFIQSLKDDPSQSANLLAEAKK
LNDAQAPKVDLKFEKEIQNAFYIILHLPNLIEEQRNAFIQSLKDDPSQSANLL
AEAKKLNDAQAPKVDLKFEKEIQNAFYIILHLPNLIEEQRNAFIQSLKDDPSQ
SANLLAEAKKLNDAQAPKVDLKFEKEIQNAFYIILHLPNLIEEQRNAFIQSLK
DDPSQSANLLAEAKKLNDAQAPKVDLKFEKEIQNAFYIILHLPNLIEEQRNA
FIQSLKDDPSQSANLLAEAKKLNDAQAPKVDLKFEKEIQNAFYIILHLPNLIE
EQRNAFIQSLKDDPSQSANLLAEAKKLNDAQAPK
SEQ ID NO: 108, Z(N3V,N6L,Q9I,E15V,N23Y)6 
VDVKFLKEIQNAFYVILHLPNLYEEQRNAFIQSLKDDPSQSANLLAEAK
KLNDAQAPKVDVKFLKEIQNAFYVILHLPNLYEEQRNAFIQSLKDDPSQSA
NLLAEAKKLNDAQAPKVDVKFLKEIQNAFYVILHLPNLYEEQRNAFIQSLK
DDPSQSANLLAEAKKLNDAQAPKVDVKFLKEIQNAFYVILHLPNLYEEQRN
AFIQSLKDDPSQSANLLAEAKKLNDAQAPKVDVKFLKEIQNAFYVILHLPNL
YEEQRNAFIQSLKDDPSQSANLLAEAKKLNDAQAPKVDVKFLKEIQNAFYV
ILHLPNLYEEQRNAFIQSLKDDPSQSANLLAEAKKLNDAQAPK
SEQ ID NO: 109, Z(N3L,N6L,Q9F,E151,N23V)6 
VDLKFLKEFQNAFYIILHLPNLVEEQRNAFIQSLKDDPSQSANLLAEAK
KLNDAQAPKVDLKFLKEFQNAFYIILHLPNLVEEQRNAFIQSLKDDPSQSAN
LLAEAKKLNDAQAPKVDLKFLKEFQNAFYIILHLPNLVEEQRNAFIQSLKDD
PSQSANLLAEAKKLNDAQAPKVDLKFLKEFQNAFYIILHLPNLVEEQRNAFI
QSLKDDPSQSANLLAEAKKLNDAQAPKVDLKFLKEFQNAFYIILHLPNLVE
EQRNAFIQSLKDDPSQSANLLAEAKKLNDAQAPKVDLKFLKEFQNAFYIILH
LPNLVEEQRNAFIQSLKDDPSQSANLLAEAKKLNDAQAPK
SEQ ID NO: 110, (B domain)4 
ADNKFNKEQQNAFYEILHLPNLNEEQRNGFIQSLKDDPSQSANLLAEA
KKLNDAQAPKADNKFNKEQQNAFYEILHLPNLNEEQRNGFIQSLKDDPSQS
ANLLAEAKKLNDAQAPKADNKFNKEQQNAFYEILHLPNLNEEQRNGFIQSL
KDDPSQSANLLAEAKKLNDAQAPKADNKFNKEQQNAFYEILHLPNLNEEQ
RNGFIQSLKDDPSQSANLLAEAKKLNDAQAPK
SEQ ID NO: 111, B(N3V)4 
ADVKFNKEQQNAFYEILHLPNLNEEQRNGFIQSLKDDPSQSANLLAEA
KKLNDAQAPKADVKFNKEQQNAFYEILHLPNLNEEQRNGFIQSLKDDPSQS
ANLLAEAKKLNDAQAPKADVKFNKEQQNAFYEILHLPNLNEEQRNGFIQSL
KDDPSQSANLLAEAKKLNDAQAPKADVKFNKEQQNAFYEILHLPNLNEEQ
RNGFIQSLKDDPSQSANLLAEAKKLNDAQAPK
SEQ ID NO: 112, B(N6E)4 
ADNKFEKEQQNAFYEILHLPNLNEEQRNGFIQSLKDDPSQSANLLAEA
KKLNDAQAPKADNKFEKEQQNAFYEILHLPNLNEEQRNGFIQSLKDDPSQS
ANLLAEAKKLNDAQAPKADNKFEKEQQNAFYEILHLPNLNEEQRNGFIQSL
KDDPSQSANLLAEAKKLNDAQAPKADNKFEKEQQNAFYEILHLPNLNEEQ
RNGFIQSLKDDPSQSANLLAEAKKLNDAQAPK
SEQ ID NO: 113, B(Q9I)4 
ADNKFNKEIQNAFYEILHLPNLNEEQRNGFIQSLKDDPSQSANLLAEAK
KLNDAQAPKADNKFNKEIQNAFYEILHLPNLNEEQRNGFIQSLKDDPSQSA
NLLAEAKKLNDAQAPKADNKFNKEIQNAFYEILHLPNLNEEQRNGFIQSLK
DDPSQSANLLAEAKKLNDAQAPKADNKFNKEIQNAFYEILHLPNLNEEQRN
GFIQSLKDDPSQSANLLAEAKKLNDAQAPK
SEQ ID NO: 114, B(Q9F)4 
ADNKFNKEFQNAFYEILHLPNLNEEQRNGFIQSLKDDPSQSANLLAEA
KKLNDAQAPKADNKFNKEFQNAFYEILHLPNLNEEQRNGFIQSLKDDPSQS
ANLLAEAKKLNDAQAPKADNKFNKEFQNAFYEILHLPNLNEEQRNGFIQSL
KDDPSQSANLLAEAKKLNDAQAPKADNKFNKEFQNAFYEILHLPNLNEEQ
RNGFIQSLKDDPSQSANLLAEAKKLNDAQAPK
SEQ ID NO: 115, B(E15L)4 
ADNKFNKEQQNAFYLILHLPNLNEEQRNGFIQSLKDDPSQSANLLAEA
KKLNDAQAPKADNKFNKEQQNAFYLILHLPNLNEEQRNGFIQSLKDDPSQS
ANLLAEAKKLNDAQAPKADNKFNKEQQNAFYLILHLPNLNEEQRNGFIQSL
KDDPSQSANLLAEAKKLNDAQAPKADNKFNKEQQNAFYLILHLPNLNEEQ
RNGFIQSLKDDPSQSANLLAEAKKLNDAQAPK
SEQ ID NO: 116, B(E15I)4 
ADNKFNKEQQNAFYIILHLPNLNEEQRNGFIQSLKDDPSQSANLLAEA
KKLNDAQAPKADNKFNKEQQNAFYIILHLPNLNEEQRNGFIQSLKDDPSQS
ANLLAEAKKLNDAQAPKADNKFNKEQQNAFYIILHLPNLNEEQRNGFIQSL
 
KDDPSQSANLLAEAKKLNDAQAPKADNKFNKEQQNAFYIILHLPNLNEEQR
NGFIQSLKDDPSQSANLLAEAKKLNDAQAPK
SEQ ID NO: 117, B(N3V,N6E,Q91,E15L)4 
ADVKFEKEIQNAFYLILHLPNLNEEQRNGFIQSLKDDPSQSANLLAEAK
KLNDAQAPKADVKFEKEIQNAFYLILHLPNLNEEQRNGFIQSLKDDPSQSAN
LLAEAKKLNDAQAPKADVKFEKEIQNAFYLILHLPNLNEEQRNGFIQSLKD
DPSQSANLLAEAKKLNDAQAPKADVKFEKEIQNAFYLILHLPNLNEEQRNG
FIQSLKDDPSQSANLLAEAKKLNDAQAPK
SEQ ID NO: 118, B(N3V) 
ADVKFNKEQQNAFYEILHLPNLNEEQRNGFIQSLKDDPSQSANLLAEA
KKLNDAQAPK
SEQ ID NO: 119, B(N6E) 
ADNKFEKEQQNAFYEILHLPNLNEEQRNGFIQSLKDDPSQSANLLAEA
KKLNDAQAPK
SEQ ID NO: 120, B(Q9I) 
ADNKFNKEIQNAFYEILHLPNLNEEQRNGFIQSLKDDPSQSANLLAEAK
KLNDAQAPK
SEQ ID NO: 121, B(Q9F) 
ADNKFNKEFQNAFYEILHLPNLNEEQRNGFIQSLKDDPSQSANLLAEA
KKLNDAQAPK
SEQ ID NO: 122, B(E15L) 
ADNKFNKEQQNAFYLILHLPNLNEEQRNGFIQSLKDDPSQSANLLAEA
KKLNDAQAPK
SEQ ID NO: 123, B(E15I) 
ADNKFNKEQQNAFYIILHLPNLNEEQRNGFIQSLKDDPSQSANLLAEA
KKLNDAQAPK
SEQ ID NO: 124, B(N3V,N6E,Q9I,E15L)

ADVKFEKEIQNAFYLILHLPNLNEEQRNGFIQSLKDDPSQSANLLAEAK

KLNDAQAPK

The invention is further described below in conjunction with specific embodiments. It should be understood that these examples are merely for illustrative purposes rather than intended to limit the scope of the disclosure. For the experimental methods without specific conditions indicated in the following examples, the conventional conditions or the conditions suggested by the manufacturer are usually followed. Percentages and parts are by weight unless otherwise indicated.

### Preparation Example 1: preparation of Z domain mutants and B domain mutants

Design the mutation site was Designed, then entire gene was synthesized by Sangon Biotech, and the mutants was expressed and purified.

During expression and purification, a single recombinant plasmid was used to transform into *Escherichia coli*, and it was fermented and inducing expressed in LB liquid medium. After fermentation, the myceliums were collected and the cell wall was destroyed by thermal lysis to release the expression product and centrifuged. The separation liquid was purified through the IgG affinity medium, loaded through the affinity medium, the medium was washed with 10 mM phosphate buffer, the target protein was collected with a buffer solution of pH 3.8, and adjusted to a neutral environment, stored for later use.

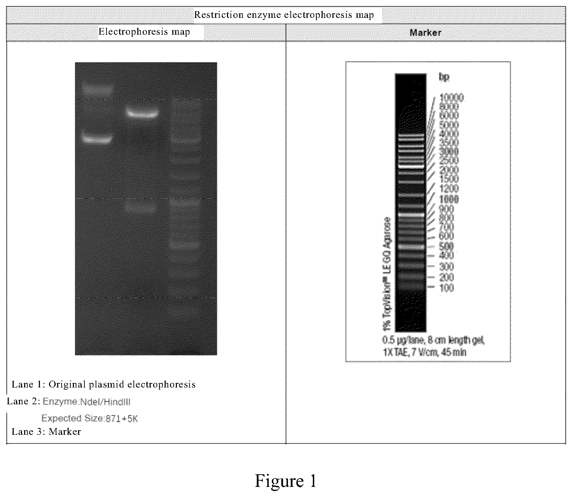

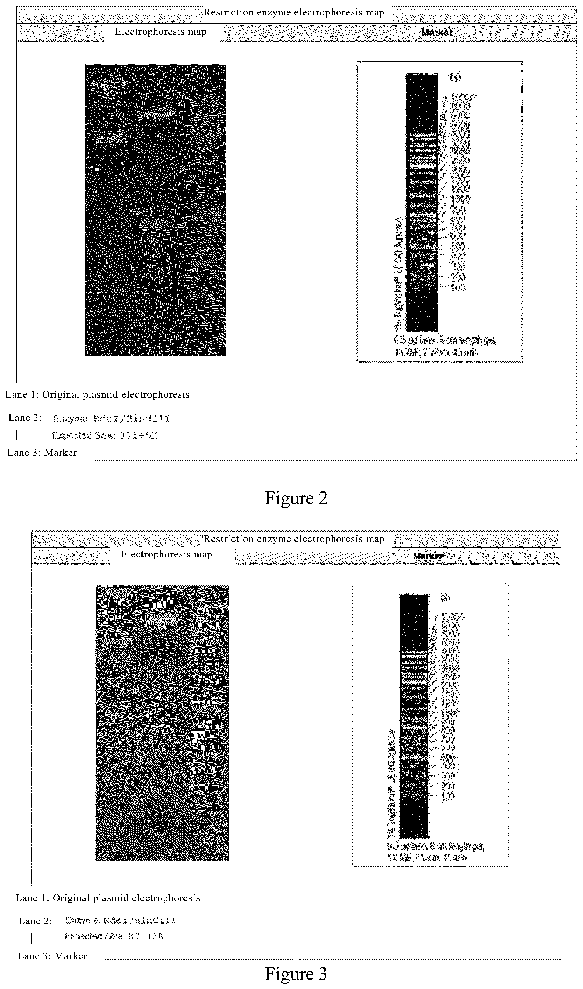

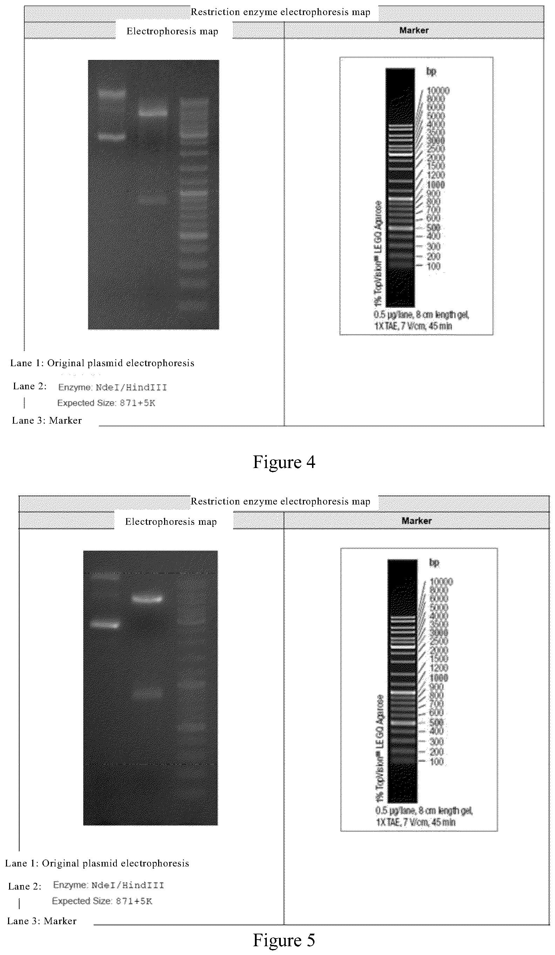

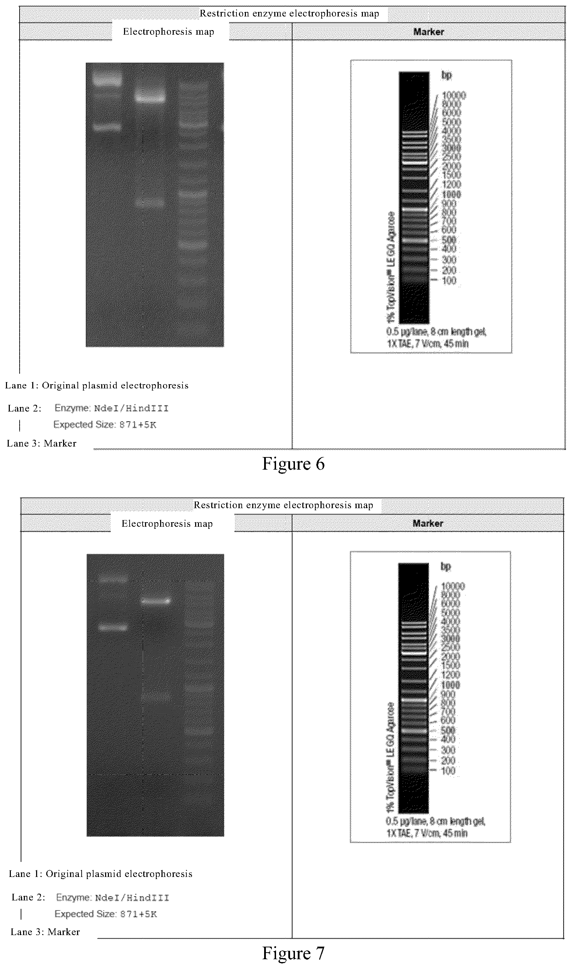

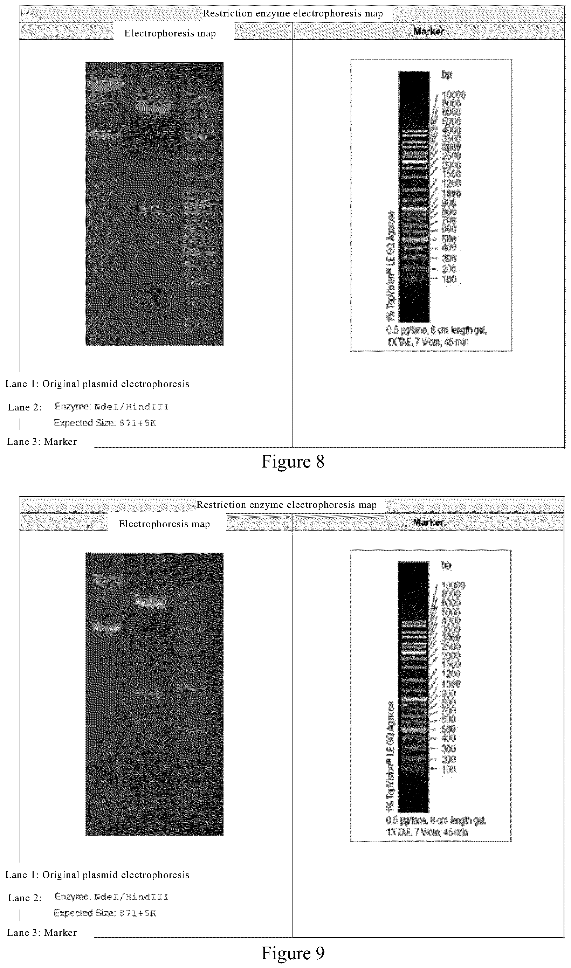

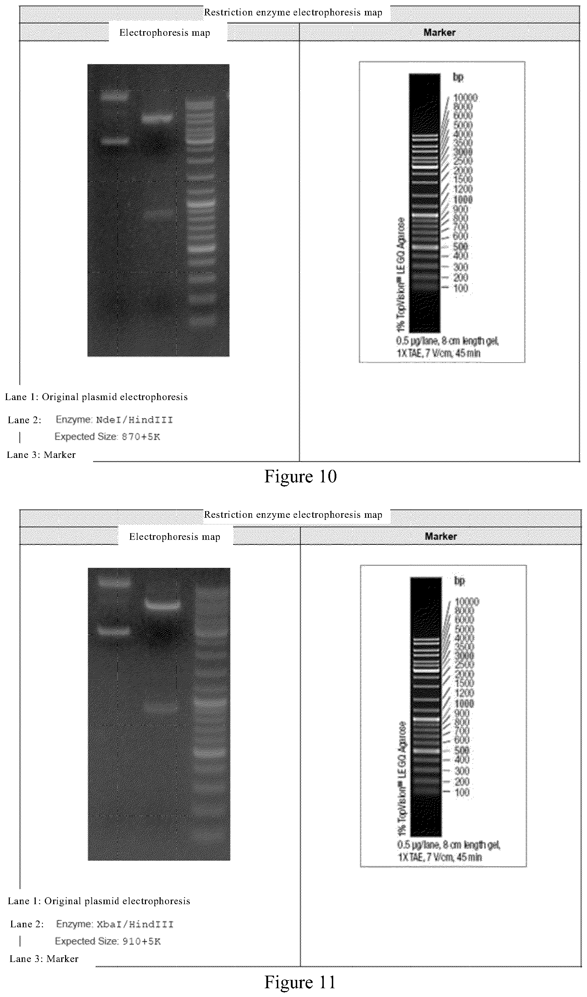

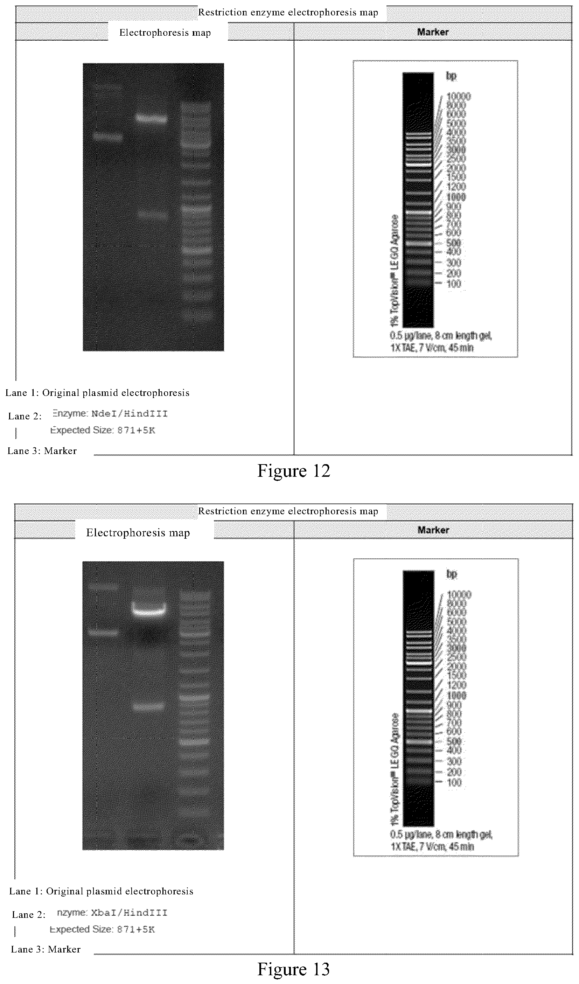

The validation analysis of the genes of some synthesized mutants are shown in Figure 1-13.

### Preparation Example 2: preparation of affinity chromatography media using Z domain mutants and B domain mutants

The obtained Z domain mutant and B domain mutant were used to prepare an affinity chromatography medium by a conventional method. An exemplary preparation process specifically includes the following steps:
a. a. Activation
   10 g of high rigidity agarose was taken, washed with purified water and drained. 10g of the above agarose was weighed, 20 mL of purified water was added, 0.2 g of sodium hydroxide and 10 mL of epichlorohydrin were added into a 100 mL flask, the above mixture was reacted at a constant temperature of 27°C for 2 hours, the activated gel was washed with 1 L of water to obtain activated agarose microspheres.
b. b. Cross-linking
   The activated agarose microspheres were added to a 100 ml falsk, then 150 mg NaHCO₃, 10 mg Na₂CO₃, 150 mg NaCl and 10 mg EDTA were added, 50 ml solution containing the mutants was added after stirring evenly, the above mixture reacted at a constant temperature of 34°C for 8 hours to complete cross-linking.
c. c. Closed
   100 mL of blocking solution (ethanolamine solution) was added to the crosslinked product, the pH was adjusted to 8.6 with sodium hydroxide, and the above mixture was reacted at a constant temperature of 26°C for 2 hours to complete the blocking of the remaining epoxy groups and reduce the impact.

### Example 1

Kinetic analysis of the Z domain mutants and B domain mutants in Table 1 were performed using the method described below.

The experimental steps are as follows:
1) Cross-link the Series S CM5 chip with IgG.
a. IgG was diluted to 5 µg/ml, 10 µg/ml, 15 µg/ml, 20 µg/ml with 10 mM acetic acid-NaOH (pH 5.0).
b. Running buffer (10 mM acetic acid-NaOH, pH5.0), desorption solution 1 (0.5% SDS), desorption solution 2 (50 mM glycine-NaOH, pH9.5), blocking solution (1 M ethanolamine pH8.5), activator (0.4 M EDC, 0.1 M NHS) were configured.
c. Run immobilization in Biacore-4000.
d. The cross-linking results are shown in the table below:

| Flow cell | Detection point | Activation method and time | Usage | Ligand concentration and name | Contact time | Unit (RU) |
|---|---|---|---|---|---|---|
| Fc-2 | 1 | Amino activation for 10 minutes | immobiliza tion | 20 µg/ml IgG | 5 min | 12300 |
| | 2 | Amino activation for 10 minutes | immobiliza tion | 15 µg/ml IgG | 5 min | 7358 |
| | 3 | Amino activation for 10 minutes | Unfixed | / | 0 | 0 |
| | 4 | Amino activation for 10 minutes | immobiliza tion | 10 µg/ml IgG | 5 min | 4285 |
| | 5 | Amino activation for 10 minutes | immobiliza tion | 5 µg/ml IgG | 5 min | 2834 |

2) Kinetic analysis
a. The mutant sample was diluted with 1XPBS (pH7.6) to 0 µg/ml, 1.615 µg/ml, 2.4375 µg/ml, 3.25 µg/ml, 4.875 µg/ml, 6.5 µg/ml, 9.75 µg/ml, 13 µg/ml, 19.5 µg/ml, 26 µg/ml.
b. Gel running buffer 1XPBS (pH7.6), regeneration solution (50 mM citric acid-NaOH pH3.0) were configured.
c. Kinetics and affinity were run in Biacore-4000.

| Contact time | Flow rate | Dissociation time | Regeneration time |
|---|---|---|---|
| 150 s | 30 µl/min | 250 s | 120 s |

The experimental results are shown in Table 1. Table 1 shows the kinetic analysis results of Z domain mutants and B domain mutants using Biacore.

**Table 1**

| Mutant | **SEQ ID NO** | Ka | Kd | KD |
|---|---|---|---|---|
| (Z domain)4 | **72** | 1.340×10⁵ | 2.971×10⁻⁴ | 2.217×10⁻⁹ |
| **Z(N3L)4** | 73 | 1.334×10⁵ | 2.346×10⁻⁴ | 1.759×10⁻⁹ |
| **Z(N3V)4** | 74 | 1.121×10⁵ | 4.509×10⁻⁴ | 4.022×10⁻⁹ |
| **Z(N6L)4** | 75 | 8.815×10⁴ | 2.693×10⁻⁴ | 3.055×10⁻⁹ |
| **Z(N6E)4** | 76 | 1.726×10⁵ | 2.752×10⁻⁴ | 1.594×10⁻⁹ |
| **Z(Q9I)4** | 77 | 1.340×10⁵ | 2.971×10⁻⁴ | 1.340×10⁵ |
| **Z(Q9F)4** | **78** | 1.296×10⁵ | 2.250×10⁻⁴ | 1.736×10⁻⁹ |
| **Z(Q9M)4** | 79 | 1.050×10⁵ | 2.584×10⁻⁴ | 2.462×10⁻⁹ |
| **Z(E15T)4** | 80 | 1.331×10⁵ | 6.263×10⁻⁴ | 4.707×10⁻⁹ |
| **Z(E15W)4** | **81** | 1.068×10⁵ | 1.646×10⁻⁴ | 1.542×10⁻⁹ |
| **Z(E15L)4** | 82 | 1.065×10⁵ | 1.330×10⁻⁴ | 1.249×10⁻⁹ |
| **Z(E15V)4** | 83 | 1.345×10⁵ | 3.573×10⁻⁴ | 2.656×10⁻⁹ |
| **Z(E15I)4** | **84** | 2.248×10⁵ | 5.732×10⁻⁴ | 2.551×10⁻⁹ |
| **Z(E15F)4** | **85** | 1.112×10⁵ | 4.440×10⁻⁴ | 3.994×10⁻⁹ |
| **Z(E15S)4** | **86** | 2.049×10⁵ | 3.651×10⁻⁴ | 1.782×10⁻⁹ |
| **Z(E15Y)4** | **87** | 1.389×10⁵ | 4.051×10⁻⁴ | 2.917×10⁻⁹ |
| **Z(E15D)4** | **88** | 2.098×10⁵ | 2.367×10⁻⁴ | 1.128×10⁻⁹ |
| **Z(N23V)4** | **89** | 9.553×10⁴ | 3.468×10⁻⁴ | 3.638×10⁻⁹ |
| **Z(N23I)4** | **90** | 7.875×10⁴ | 4.614×10⁻⁴ | 5.859×10⁻⁹ |
| **Z(N23Y)4** | **91** | 1.718×10⁵ | 2.544×10⁻⁴ | 1.481×10⁻⁹ |
| **Z(N3L,N6L,Q9I,E15L)4** | **95** | 1.689×10⁵ | 3.535×10⁻⁴ | 2.093×10⁻⁹ |
| **Z(N3V,N6E,Q9I,E15L)4** | **97** | 1.380×10⁵ | 3.752×10⁻⁴ | 2.720×10⁻⁹ |
| **Z(N3L,Q9I,E15L,N23V)4** | **98** | 1.271×10⁵ | 5.610×10⁻⁴ | 4.414×10⁻⁹ |
| **Z(N6L,Q9F,E15I,N23V)4** | **99** | 1.135×10⁵ | 4.541×10⁻⁴ | 3.977×10⁻⁹ |
| **Z(N3L,N6L,Q9I,E15L,N23V)4** | **100** | 1.322×10⁵ | 3.083×10⁻⁴ | 2.331×10⁻⁹ |
| **Z(N3L,N6E,Q9I,E15I,N23I)4** | **101** | 6.995×10⁴ | 1.796×10⁻⁴ | 2.567×10⁻⁹ |
| **Z(N3V,N6L,Q9F,E15V,N23Y)4** | **102** | 9.564×10⁴ | 2.641×10⁻⁴ | 2.762×10⁻⁹ |
| (B domain)4 | 110 | 1.003×10⁵ | 2.861×10⁻⁴ | 2.852×10⁻⁹ |
| **B(N3V)4** | 111 | 7.052×10⁴ | 2.921×10⁻⁴ | 4.142×10⁻⁹ |
| **B(N6E)4** | 112 | 7.656×10⁴ | 1.655×10⁻⁴ | 2.162×10⁻⁹ |
| **B(Q9I)4** | 113 | 1.802×10⁵ | 5.513×10⁻⁴ | 3.306×10⁻⁹ |
| **B(Q9F)4** | **114** | 8.360×10⁴ | 1.842×10⁻⁴ | 2.203×10⁻⁹ |
| **B(E15L)4** | 115 | 1.513×10⁵ | 3.647×10⁻⁴ | 2.410×10⁻⁹ |
| **B(E15I)4** | **116** | 1.083×10⁵ | 3.614×10⁻⁴ | 3.337×10⁻⁹ |
| **B(N3V,N6E,Q9I,E15L)4** | **117** | 1.583×10⁵ | 2.101×10⁻⁴ | 1.328×10⁻⁹ |

The results showed that compared with the corresponding Z or B domains, the KD values of each mutant were basically on the same order of magnitude, and the dissociation constant did not change significantly. This indicates that the binding ability to IgG of the mutant obtained by mutating the Z and B domains in the present description has not been affected .

### Example 2

The IgG binding ability and alkali resistance stability of the Z domain mutant and B domain mutant affinity chromatography medium in Table 2 were evaluated using the method described below.

The experimental steps are as follows:
1) 1) 4.4mL of the affinity chromatography medium of Z domain mutant or B domain mutant was taken and packed into the chromatography column with 20% ethanol at a flow rate of 5 ml/min.
Buffers: buffer A (PBS, pH 7.6); buffer B (0.1M Sodium Citrate, pH 3.0); buffer C (1M NaOH).
Sample: 1 mg/ml of IgG sample was prepared with the pure product (the solution for diluting the pure product is buffer A).

2) 2) dynamic binding capacity @ 10% breakthrough determination:
Equipment: AKTA Pure.
Determination process:
   a. a. Buffer A equilibrated chromatography column, flow rate: 1.8 ml/min;
   b. b. When the IGg sample does not flow through the column, its UV absorption peak was detected, calculated as λₘₐₓ, and 10% λₘₐₓ was calculated accordingly;
   c. c. Sample loading: flow rate: 1.8 ml/min, the UV absorption peak of the basic flow through was recorded;
   d. d. The two was added to get the UV absorption peak corresponding to dynamic binding capacity @ 10% breakthrough;
   e. e. Keep loading the sample until the UV absorption peak reaches the volume corresponding to the UV absorption peak in step d, which is dynamic binding capacity @ 10% breakthrough.

3) 3) Alkali resistance cycle:
The room temperature was controlled at 23±0.5°C; the sample loading volume in the program was updated according to the decrease of the binding capacity, and the other programs were kept consistent.
Determination process:
   a. a. PBS equilibration: flow rate: 1.8 ml/min; volume: 1 cv;
   b. b. Sample loading: flow rate: 1.8 ml/min; 200 ml (in subsequent procedures, the sample volume was updated according to the decline of the binding capacity);
   c. c. PBS cleaning: flow rate: 1.8 ml/min; volume: 5 cv;
   d. d.0.1M sodium citrate elution: flow rate: 1.8 ml/min; volume: 3 cv;
   e. e. Process with 1M NaOH for 2 hours: first using 1.8 ml/min flow rate to pass 1 cv to fill the chromatography column with 1M NaOH, then change to flow rate: 0.5 ml/min; volume: 60 ml; time: 2 h;
   f. f. PBS equilibration: flow rate: 1.8 ml/min; volume: 10 cv;

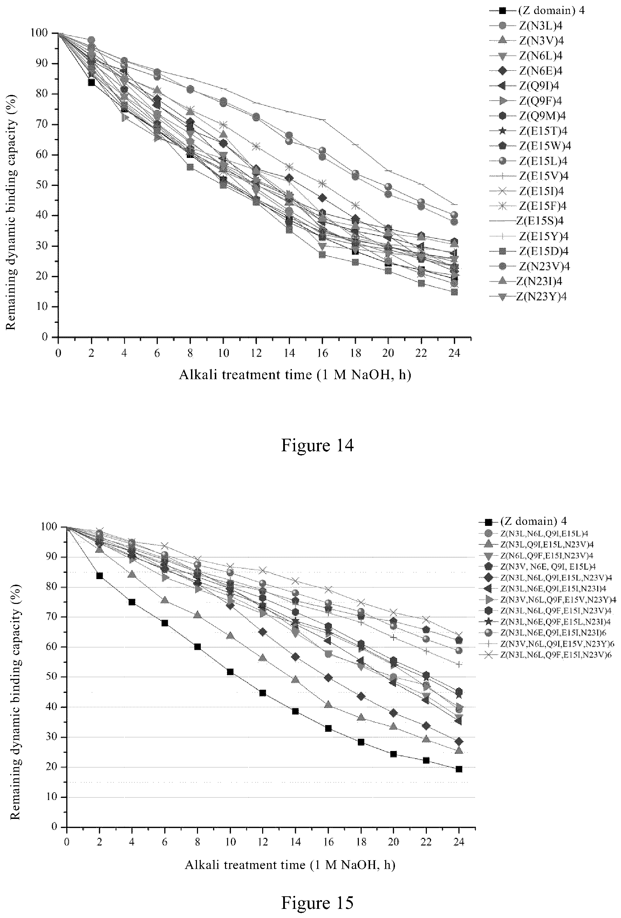

₄₎ 4) The above-mentioned alkali treatment procedure was carried out 13 times, the corresponding binding capacity was recorded, and a chart was drawn. The results were shown in Table 2 and Figure 14, 15 (Z(N3VN6E,Q91,E15L)4) and 16. Table 2 shows the DBC of IgG (in terms of dynamic binding capacity, mg/ml) at 10% breakthrough (Qb10%) for each sample.

**Table 2**

| **Mutant** | **SEQ ID NO** | **Initial IgG capability (mg/ml)** | **Remaining IgG capacity after 24 hours (mg/ml)** | **Remaining IgG capacity after 24 hours (%)** |
|---|---|---|---|---|
| **(Z domain)4** | **72** | **173.29** | **33.41** | **19.28** |
| Z(N3L)4 | 73 | 190.45 | 33.73 | **17.71** |
| **Z(N3V)4** | 74 | **155.94** | **36.66** | **23.51** |
| **Z(N6L)4** | 75 | 133.76 | 31.09 | **23.24** |
| **Z(N6E)4** | 76 | **165.35** | **35.89** | **21.71** |
| **Z(Q9I)4** | 77 | **143.25** | **39.56** | **27.62** |
| **Z(Q9F)4** | **78** | **181.77** | **37.17** | **20.45** |
| **Z(Q9M)4** | 79 | **135.52** | **42.65** | **31.47** |
| **Z(E15T)4** | 80 | **175.64** | **45.53** | **25.92** |
| **Z(E15W)4** | **81** | **164.57** | **38.14** | **23.18** |
| **Z(E15L)4** | 82 | **157.28** | **63.05** | **40.08** |
| **Z(E15V)4** | 83 | **133.35** | **33.16** | **24.87** |
| **Z(E15I)4** | **84** | **186.51** | **33.01** | **17.70** |
| **Z(E15F)4** | **85** | **155.32** | **35.47** | **22.84** |
| **Z(E15S)4** | **86** | **165.33** | **72.03** | **43.57** |
| **Z(E15Y)4** | **87** | **173.63** | **48.61** | **27.99** |
| **Z(E15D)4** | **88** | **145.09** | **21.56** | **14.86** |
| **Z(N23V)4** | **89** | **149.85** | **56.72** | **37.85** |
| **Z(N23I)4** | **90** | **176.54** | **53.91** | **30.54** |
| **Z(N23Y)4** | **91** | **166.17** | **43.02** | **25.89** |
| **Z(N3V,N6E,Q9I,E15L)4** | **97** | **176.90** | **109.98** | **62.17** |
| (B domain)4 | **110** | **129.41** | **21.14** | **16.34** |
| **B(N3V)4** | **111** | 139.31 | **37.12** | **26.65** |
| **B(N6E)4** | **112** | **133.50** | **39.18** | **29.35** |
| **B(Q9I)4** | **113** | 179.09 | 70.18 | **39.19** |
| **B(Q9F)4** | **114** | **139.78** | **42.60** | **30.84** |
| **B(E15L)4** | **115** | **171.10** | **85.77** | **50.13** |
| **B(E15I)4** | **116** | **123.50** | **54.12** | **43.82** |
| **B(N3V,N6E,Q9I,E15L)4** | **117** | **151.33** | **92.99** | **61.45** |

The results showed that the initial IgG binding ability of the mutants was not significantly different from that of the Z domain. The initial IgG binding ability of the mutants in the table was all above 130 mg/ml, indicating good affinity. After 24 hours of alkaline treatment with 1.0 M NaOH, except for a small number of mutants that showed slightly worse IgG binding ability than the Z domain, the IgG binding ability of most mutants was better than that of the Z and B domains. Among them, mutant Z (E15S) 4 still had 43.57% of residual IgG binding capacity after 24 hours of alkaline treatment, indicating that the selected mutation site of the present description can improve the alkaline stability of the Z and B domains.

### Example 3

The affinity chromatography medium in table 3 was obtained by Z domain, which was processed repeatedly by approach in Example 2, and the results were shown in Table 3 and FIG. 15. Table 3 shows the DBC of IgG (in terms of dynamic binding capacity, mg/ml) at 10% breakthrough (Qb10%) for each sample.

**Table 3**

| Z domain mutant | SEQ ID NO | Initial IgG capability (mg/ml) | Remaining IgG capacity after 24 hours (mg/ml) | Remaining IgG capacity after 24 hours (%) |
|---|---|---|---|---|
| (Z domain)4 | 72 | 173.29 | 33.41 | 19.28 |
| Z(N3L,N6L,Q9I,E15L)4 | 95 | 179.09 | 70.18 | 39.19 |
| Z(N3L,Q9I,E15L,N23V)4 | 98 | 165.88 | 42.06 | 25.36 |
| Z(N6L,Q9F,E15I,N23V)4 | 99 | 185.57 | 68.17 | 36.74 |
| Z(N3L,N6L,Q9I,E15L,N23V )4 | 100 | 158.7 | 45.27 | 28.53 |
| Z(N3V,N6L,Q9F,E15V,N23 Y)4 | 102 | 169.28 | 67.89 | 40.11 |
| Z(N3L,N6E,Q9I,E15I,N23I) 4 | 101 | 161.31 | 57.17 | 35.44 |
| Z(N3L,N6L,Q9F,E15I,N23V )4 | 104 | 151.22 | 68.37 | 45.21 |
| Z(N3L,N6E,Q9F,E15L,N23I )4 | 105 | 147.58 | 64.97 | 44.02 |
| Z(N3L,N6E,Q9I,E15I,N23I) 6 | 107 | 149.35 | 87.81 | 58.79 |
| Z(N3V,N6L,Q9I,E15V,N23 Y)6 | 108 | 190.17 | 103.07 | 54.20 |
| Z(N3L,N6L,Q9F,E15I,N23V )6 | 109 | 174.67 | 111.67 | 63.93 |

Table 3 shows the alkali tolerance of mutants with multiple site mutations in the Z domain. After 24 hours of treatment with 1.0 M NaOH, the residual IgG binding ability of the mutants was improved compared to the Z domain, indicating that the above mutations improved alkali tolerance. Among them, mutant Z (N3L, N6L, Q9F, E151, N23V)6 still present 63.93% IgG binding capacity after 24 hours of alkaline treatment.

## Claims

1. A separated polypeptide, wherein, the polypeptide is selected from:
(1) a polypeptide containing substitution mutation at one or more positions selected from positions 3, 6, 9, 15 and 23 compared with the native B domain of protein A shown in SEQ ID NO: 1 or the Z domain of protein A shown in SEQ ID NO: 2,
wherein, the substitution mutation at position 3 is that asparagine is replaced by leucine, isoleucine, valine or tyrosine; the substitution mutation at position 6 is that asparagine is replaced by glutamic acid, leucine, isoleucine, valine, serine or threonine; the substitution mutation at position 9 is that glutamine is replaced by isoleucine, leucine, valine, phenylalanine or methionine; the substitution mutation at position 15 is that glutamic acid is replaced by threonine, tryptophan, leucine, valine, isoleucine, phenylalanine, serine, tyrosine or aspartic acid; the substitution mutation at position 23 is that asparagine is replaced by valine, isoleucine, leucine or tyrosine;
(2) a polypeptide having at least 85% identity with the polypeptide described in (1) and retaining the substitution mutation at one or more positions selected from positions 3, 6, 9, 15 and 23.

2. The polypeptide according to claim 1, wherein,
the substitution mutation at position 3 is that asparagine is replaced by leucine, valine or tyrosine;
the substitution mutation at position 6 is that asparagine is replaced by glutamate, leucine or serine;
the substitution mutation at position 9 is that glutamine is replaced by isoleucine, methionine or phenylalanine;
the substitution mutation at position 15 is that glutamate is replaced by threonine, tryptophan, leucine, isoleucine, valine, phenylalanine, serine or tyrosine;
the substitution mutation at position 23 is that asparagine is mutated to valine, isoleucine or tyrosine.

3. The polypeptide according to claim 1 or 2, wherein, the polypeptide:
has at least the substitution mutation at position 3, and has the substitution mutation at least at one position, at least at two positions, at least at three positions, or at all four positions selected from positions 15, 6, 9, and 23;
has at least the substitution mutation at position 6, and has the substitution mutation at least at one position, at least at two positions, at least at three positions, or at all four positions selected from positions 3, 15, 9, and 23;
has at least the substitution mutation at position 9, and has the substitution mutation at least at one position, at least at two positions, at least at three positions, or at all four positions selected from positions 3, 6, 15, and 23;
has at least the substitution mutation at position 15, and has the substitution mutation at least at one position, at least at two positions, at least at three positions, or at all four positions selected from positions 3, 6, 9, and 23; or
has at least the substitution mutation at position 23, and has the substitution mutation at least at one position, at least at two positions, at least at three positions, or at all four positions selected from positions 3, 6, 9, and 15;
preferably, the substitution mutation at position 15 is E15I, E15L or E15V, the substitution mutation at position 3 is N3L or N3V, the substitution mutation at position 6 is N6E or N6L, and the substitution mutation at position 9 is Q9I or Q9F, the substitution mutation at position 23 is N23I, N23Y or N23V.

4. The polypeptide according to claim 1 or 2, wherein the polypeptide has the substitution mutation at positions 3, 9 and 15, and has the substitution mutation at positions 6 and/or 23;
preferably, the substitution mutation at position 3 is N3L or N3V, the substitution mutation at position 6 is N6L or N6E, the substitution mutation at position 9 is Q9I or Q9F, and the substitution mutation at position 15 is E15L, E15I or E15V, the substitution mutation at position 23 is N23V, N23I or N23Y.

5. The polypeptide according to claim 1, wherein the amino acid sequence of the polypeptide is as shown in any one of SEQ ID NO: 3-71 and SEQ ID NO: 118-124.

6. A separated polypeptide, wherein, the polypeptide consists of the following (i), (ii) and (iii): (i) the polypeptide according to any one of claims 1 to 5, (ii) one or more coupling elements at the C-terminus or N-terminus of the amino acid sequence of the peptide (i), and optionally (iii) residues from the excised signal transduction sequence.

7. The polypeptide according to claim 6, wherein the coupling element is selected from the group consisting of: cysteine residues, multiple lysine residues and multiple histidine residues; the residues from the excised signal transduction sequence are AQ.

8. A fusion protein, wherein the fusion protein comprises an amino acid sequence formed by fusion of 2-8 polypeptides according to any one of claims 1 to 5, wherein the 2-8 polypeptides are different from each other, partially or completely identical, and optionally there is a linker sequence between the polypeptides.

9. The fusion protein according to claim 8, wherein, among the 2-8 polypeptides contained in the fusion protein, at least one polypeptide has the substitution mutation at position 15, optionally has the substitution mutation at least at one position, at least at two positions, at least at three positions or at all four positions selected from positions 3, 6, 9 and 23; preferably, at least one polypeptide has the substitution mutation at positions 3, 9 and 15, and has the substitution mutation at positions 6 and/or 23.

10. The fusion protein according to claim 8, wherein, in the fusion protein, the polypeptide is selected from one or more of: SEQ ID NO: 4, SEQ ID NO: 8, SEQ ID NO: 9, SEQ ID NO: 10, SEQ ID NO: 11, SEQ ID NO: 14, SEQ ID NO: 16, SEQ ID NO: 18, SEQ ID NO: 21, SEQ ID NO: 22, SEQ ID NO: 51, SEQ ID NO: 53, SEQ ID NO: 60, SEQ ID NO: 71, SEQ ID NO: 64, SEQ ID NO: 69, SEQ ID NO: 70, SEQ ID NO: 68, SEQ ID NO: 118, SEQ ID NO: 119, SEQ ID NO: 120, SEQ ID NO: 121, SEQ ID NO: 122, SEQ ID NO: 123, and SEQ ID NO:124.

11. The fusion protein according to any one of claims 8 to 10, wherein the fusion protein further comprises at the C-terminus or N-terminus one or more coupling elements and/or residues from the excised signal transduction sequence; preferably, the coupling element is selected from the group consisting of: cysteine residues, multiple lysine residues and multiple histidine residues.

12. The fusion protein according to claim 8, wherein the amino acid sequence of the fusion protein is as shown in any one of SEQ ID NO: 73-109 and SEQ ID NO: 110-117.

13. A recombinant protein A, the B domain of the recombinant protein A is the polypeptide according to any one of claims 1 to 5.

14. A separated nucleic acid molecule, the polynucleotide sequence of the nucleic acid molecule is selected from the group consisting of:
(1) a polynucleotide sequence encoding the polypeptide according to any one of claims 1 to 7, the fusion protein according to any one of claims 8 to 12 or the recombinant protein A according to claim 13;
(2) the complementary sequence of the polynucleotide sequence of (1).

15. A nucleic acid construct, wherein, the nucleic acid construct comprises the nucleic acid molecule according to claim 14; preferably, the nucleic acid construct is an expression cassette; more preferably, the nucleic acid construct is an expression vector or cloning vector.

16. An expression system comprising the nucleic acid construct according to claim 15; preferably, the expression system is a host cell.

17. A separation medium, wherein the separation medium comprises the polypeptide according to any one of claims 1 to 7, the fusion protein according to any one of claims 8 to 12 and/or the recombinant protein A according to claim 13 coupled to a solid support.

18. The separation medium according to claim 17, wherein the polypeptide, the fusion protein or the recombinant protein A is coupled to the solid support through a thioether bond.

19. The separation medium according to claim 18, wherein the solid support is selected from:
a polymer containing polyhydroxyl groups, preferably polysaccharides, more preferably selected from: dextran, starch, cellulose, pullulan, agar and agarose;
a synthetic polymer, preferably selected from the group consisting of: polyvinyl alcohol, polystyrene, polystyrene divinylbenzene, polyhydroxyalkyl acrylates, polyhydroxyalkyl methacrylates, polyacrylamides and polymethacrylamides; and
a support of inorganic nature, preferably selected from silica and zirconia.

20. A chromatographic column, wherein the chromatographic column comprises the separation medium according to any one of claims 17 to 19.

21. A method for separating an Fc-containing protein, wherein the method comprises a step where bring an immunoglobulin-containing sample comes in contact with the polypeptide according to any one of claims 1 to 7, a fusion protein according to any one of claims 8 to 12, a recombinant protein A according to claim 13, a separation medium according to any one of claims 17 to 19, or a chromatographic column according to claim 20; preferably, the Fc-containing protein is immunoglobulin.

22. The method according to claim 21, wherein, the method comprise:
(1) bringing the sample comprises an Fc-containing protein into contact with the separation medium ;
(2) washing the separation medium;
(3) eluting the Fc-containing protein from the separation medium ;
(4) washing the separation medium;
preferably, washing the separation medium with 0.1-2.0 M or 0.5-1.0 M NaOH or KOH solution.

23. The use of the polypeptide according to any one of claims 1 to 7, the fusion protein according to any one of claims 8 to 12, the recombinant protein A according to claim 13 in the separation of Fc-containing proteins, or in the preparation of a separation medium or chromatographic column for separating Fc-containing proteins.
